# EUROPEAN PATENT SPECIFICATION

(11) **EP 4 173 606 B1**
(45) Date of publication and mention of the grant of the patent: **11.03.2026**
(21) Application number: 21875812.6
(22) Date of filing: 30.09.2021
(51) Int. Cl.: A61F 13/49, A61F 13/513

(54) **PANT-TYPE ABSORBENT ARTICLE**
HOSENARTIGER SAUGFÄHIGER ARTIKEL
ARTICLE ABSORBANT DE TYPE CULOTTE

(30) Priority: 30.09.2020 CN 202011059253
(43) Date of publication of application: 03.05.2023
(73) Proprietor: Unicharm Corporation, Ehime 799-0111 (JP)
(72) Inventor: KAWAKAMI, Yusuke, Kanonji-shi, Kagawa 769-1602 (JP); GAO, Juyi, Shanghai 201700 (CN); WANG, Yinhua, Shanghai 201700 (CN); ZHENG, Lingshuang, Shanghai 201700 (CN)
(74) Representative: Dolleymores
(86) International application number: PCT/JP2021/036270
(87) International publication number: WO 2022/071516

(56) References cited:
- WO-A1-2019/169989
- WO-A1-2019/169989
- WO-A1-2020/026683
- WO-A1-2020/164379
- JP-A- 2017 012 319
- JP-A- 2017 012 319
- JP-A- 2017 113 186
- JP-A- 2020 018 388
- JP-A- 2020 080 909
- JP-A- 2020 080 909
- JP-A- 2020 127 539
- JP-A- 2020 151 352
- JP-B1- 6 605 167

## Description

### FIELD

The present invention relates to an underpants-shaped absorbent article.

### BACKGROUND

Conventionally, in an absorbent article such as an underpants-shaped disposable diaper, there has been known a technique for increasing water absorbency by using a hydrophilic sheet for a part of a waist member. For example, Patent Literature 1 discloses a disposable diaper in which a sheet member such as a nonwoven fabric containing hydrophilic fibers is used in a skin-side sheet 5 located on the skin side of a wearer and an outer-layer sheet 4 located on the non-skin side.

### [CITATION LIST]

### [PATENT LITERATURE]

Patent Literature 1: Japanese Patent Application Publication No. 2017-113186
JP 2017 012319 A and WO 2020/164379 A1 disclose prior art examples of absorbent articles comprising waist members having a nonwoven fabric containing hydrophobic fibers on a skin side and a nonwoven fabric containing hydrophilic fibers on a non-skin side.

### SUMMARY

### [TECHNICAL PROBLEM]

Generally, in an underpants-shaped diaper formed of nonwoven fabric such as that described in Patent Literature 1, heat is likely to be trapped between the wearer's body and the diaper compared with fabric underwear or the like, and there is a risk that the internal temperature rises while putting on the diaper, causing stuffiness inside the underpants-shaped diaper (absorbent article) or making the wearer feel discomfort.

In addition, in a diaper using a hydrophilic sheet member, it can make it likely to absorb sweat and other moisture from the wearer's skin. However, even when moisture is absorbed from the wearer's skin by the hydrophilic sheet, there is a problem that moisture retained in the hydrophilic sheet is less likely to be evaporated. In this case, there is a risk that the hydrophilic sheet containing moisture comes into contact with the wearer's skin, to make the wearer feel discomfort.

The present invention was achieved in light of conventional problems such as that described above and an aspect of the present invention is to provide an underpants-shaped absorbent article capable of making a wearer less likely to feel discomfort. More specifically, the first problem is to provide an underpants-shaped absorbent article whose internal temperature is less likely to rise while being put on, and the second problem is to provide an underpants-shaped absorbent article capable of absorbing moisture from the wearer's skin and efficiently evaporating the absorbed moisture into the atmosphere.

### [SOLUTION TO PROBLEM]

A main aspect of the present invention for solving the second problem is an underpants-shaped absorbent article having a vertical direction, a lateral direction, and a front-back direction that intersect with each other, the underpants-shaped absorbent article including: a liquid-absorbent absorbent main body; and a waist member that is provided on a non-skin side with respect to the absorbent main body, an at least partial region of the waist member having a hydrophobic nonwoven fabric that is arranged farthest on a skin side, a hydrophilic nonwoven fabric that is overlaid adjacent to a non-skin side of the hydrophobic nonwoven fabric and that has higher hydrophilicity than the hydrophobic nonwoven fabric, and an elastic member that is provided between the hydrophobic nonwoven fabric and the hydrophilic nonwoven fabric and that stretches and contracts in the lateral direction.
Features of the present invention other than the above will become clear by reading the description of the present specification with reference to the accompanying drawings.

### [ADVANTAGEOUS EFFECT OF THE INVENTION]

An effect of the present invention on the second problem is to enable to provide an underpants-shaped absorbent article capable of absorbing moisture from the wearer's skin and efficiently evaporating the absorbed moisture into the atmosphere.

### BRIEF DESCRIPTION OF DRAWINGS

FIG. 1 is a schematic perspective view of a diaper 1.
FIG. 2 is a plan view of the diaper 1 in an unfolded and stretched state.
FIG. 3 is a schematic cross-sectional view taken along a line A-A in FIG. 2.
FIG. 4A is a plan view of an absorbent main body 10, and FIG. 4B is a schematic cross-sectional view of the absorbent main body 10.
FIGS. 5A and 5B are diagrams illustrating a cross-sectional shape of a front waist portion 30 while the diaper 1 is put on.
FIG. 6 is a cross-sectional view showing a region X in FIG. 5A in an enlarged manner.
FIG. 7 is a cross-sectional view showing a region Y in FIG. 5B in an enlarged manner.
FIG. 8 is a cross-sectional view showing the region Y in FIG. 5B in an enlarged manner, and shows a state after a predetermined time has elapsed from a state shown in FIG. 7.
FIG. 9 is an explanatory diagram illustrating evaporation of moisture in the case where hole portions 32h are provided in a non-skin-side sheet 32.
FIGS. 10A and 10B are explanatory diagrams illustrating a method for attaching a waist elastic member 35 to the front waist portion 30 using welding portions 60.
FIG. 11A is an explanatory diagram illustrating the temperature measurement position on the front side of a sweating mannequin, and FIG. 11B is an explanatory diagram illustrating the temperature measurement position on the back side of the sweating mannequin.
FIGS. 12A and 12B are diagrams showing the results of measurement of the inside-underpants temperature for several types of underpants-shaped wearing article.
FIG. 13A is a plan view of the diaper 2 in an unfolded and stretched state.
FIG. 13B is a schematic cross-sectional view taken along a line B-B in FIG. 13A.
FIG. 14A is a plan view of a diaper 3 in an unfolded and stretched state.
FIG. 14B is a schematic cross-sectional view taken along a line C-C in FIG. 14A.

### DESCRIPTION OF EMBODIMENTS

At least following matters will become clear with description of this specification and attached drawings.

An underpants-shaped absorbent article including: an absorbent main body; and a waist member having a hydrophobic nonwoven fabric and a hydrophilic nonwoven fabric, the hydrophilic nonwoven fabric being located on a non-skin side with respect to the hydrophobic nonwoven fabric and having higher hydrophilicity than the hydrophobic nonwoven fabric, in an inside-underpants-temperature measuring experiment using a sweating mannequin that has a temperature sensor at a predetermined position, in a state where the underpants-shaped absorbent article is put on the sweating mannequin so as to cover a predetermined position, letting a temperature measured at the predetermined position be a first temperature, and in a state where the predetermined position is not covered, letting a temperature measured at the predetermined position be a second temperature, the hydrophilic nonwoven fabric being arranged in the waist member in a manner such that a difference between the first temperature and the second temperature is 1.5°C or less after a lapse of 60 minutes from start of power supply to the sweating mannequin.

According to the above-described underpants-shaped absorbent article, moisture absorbed by the hydrophilic nonwoven fabric is evaporated from the waist member into the atmosphere. This makes it more likely to remove heat of vaporization, making it possible to keep 1.5°C or less the difference between the inside-underpants temperature in a putting state of the underpants-shaped absorbent article and the temperature in a non-putting state. That is, heat is less likely to be trapped inside the underpants-shaped absorbent article, and an excessive temperature rise is suppressed. This can make the wearer less likely to feel discomfort.

In such an underpants-shaped absorbent article, it is desirable that in the inside-underpants-temperature measuring experiment, in a state where an underpants-shaped absorbent article including a waist member in which the hydrophilic nonwoven fabric is not provided is put on the sweating mannequin so as to cover the predetermined position, letting a temperature measured at the predetermined position be a third temperature, the hydrophilic nonwoven fabric is arranged in the waist member in a manner such that a difference between the third temperature and the first temperature is 1°C or more after a lapse of 60 minutes from start of power supply to the sweating mannequin.

According to the above-described underpants-shaped absorbent article, compared with an underpants-shaped absorbent article in which the hydrophilic nonwoven fabric is not used, the temperature rise after 60 minutes have elapsed can be lowered by 1°C or more. That is, compared with a conventional underpants-shaped absorbent article formed of a hydrophobic nonwoven fabric, it can make the temperature rise while putting on the absorbent article likely to be suppressed. This can make the wearer less likely to feel discomfort.

In such an underpants-shaped absorbent article, it is desirable that in the inside-underpants-temperature measuring experiment, the hydrophilic nonwoven fabric is arranged in the waist member in a manner such that a temperature rise rate per unit time of the first temperature for 30 minutes after the start of the temperature rise rate per unit time of the third temperature for 30 minutes after the start of the power supply to the sweating mannequin.

According to the above-described underpants-shaped absorbent article, compared with an underpants-shaped absorbent article in which the hydrophilic nonwoven fabric is not used, the temperature rise rate after 30 minutes has elapsed can be suppressed to 1/2 or less. That is, it is possible to realize an underpants-shaped absorbent article in which the temperature rise rate immediately after the absorbent article is put on (for 30 minutes) is kept low compared with a conventional underpants-shaped absorbent article formed of a hydrophobic nonwoven fabric, and which has a excellent feeling of use. This can make the wearer less likely to feel discomfort.

In such an underpants-shaped absorbent article, it is desirable that the waist member has a front waist portion and a back waist portion, and the predetermined position is a position covered by at least one of the front waist portion and the back waist portion in a state where the underpants-shaped absorbent article is put on the sweating mannequin.

According to the above-described underpants-shaped absorbent article, the temperature rise at a position where the wearer's body is covered with the waist member during usage is likely to be suppressed. That is, in the wearer's front and back waist regions in which the wearer is more likely to sweat, it makes it easier to suppress the inside-underpants temperature being excessively high and stuffiness in the underpants. This can make the wearer less likely to feel discomfort.

Further, it becomes clear that an underpants-shaped absorbent article in accordance with the present invention has a vertical direction, a lateral direction, and a front-back direction that intersect with each other, the underpants-shaped body; and a waist member that is provided on a non-skin side with respect to the absorbent main body, an at least partial region of the waist member having a hydrophobic nonwoven fabric that is arranged farthest on a skin side, a hydrophilic nonwoven fabric that is overlaid adjacent to a non-skin side of the hydrophobic nonwoven fabric and that has higher hydrophilicity than the hydrophobic nonwoven fabric, and an elastic member that is provided between the hydrophobic nonwoven fabric and the hydrophilic nonwoven fabric and that stretches and contracts in the lateral direction, wherein an average fiber density of the hydrophobic nonwoven fabric is larger than an average fiber density of the hydrophilic nonwoven fabric.

According to the above-described underpants-shaped absorbent article, accompanying with the contraction of the elastic member, the hydrophobic nonwoven fabric contracts, and thereby the inter-fiber distance between the fibers that constitute the hydrophobic nonwoven fabric is reduced. This causes a capillary phenomenon and makes it likely to absorb moisture from the wearer's body. Then, accompanying with the stretch of the elastic member, the hydrophobic nonwoven fabric and the hydrophilic nonwoven fabric stretch and becomes likely to come into surface contact with each other. Consequently, the moisture absorbed by the hydrophobic nonwoven fabric is transferred to the hydrophilic nonwoven fabric side (non-skin side) and diffused in the hydrophilic nonwoven fabric, evaporating the moisture to the outside (non-skin side) from the non-skin side surface of the hydrophilic nonwoven fabric. Accordingly, it is possible to realize an underpants-shaped absorbent article capable of absorbing moisture from the wearer's skin and efficiently evaporating the absorbed moisture into the atmosphere.

In such an underpants-shaped absorbent article, it is desirable that a portion in which the hydrophobic nonwoven fabric and the hydrophilic nonwoven fabric are joined is provided around at least the elastic member.

According to the above-described underpants-shaped absorbent article, due to an influence of the stretching and contraction of the elastic member, the hydrophobic nonwoven fabric and the hydrophilic nonwoven fabric are likely to stretch and contract, this promotes the movement of moisture. Further, since the sheets are in close contact with each other at the joining portion, the moisture is likely to be smoothly transferred from the hydrophobic nonwoven fabric to the hydrophilic nonwoven fabric. Accordingly, in the absorbent article, moisture is likely to be evaporated, and this can make the wearer less likely to feel discomfort.

In such an underpants-shaped absorbent article, it is desirable that the hydrophobic nonwoven fabric and the hydrophilic nonwoven fabric are joined with an adhesive via the elastic member.

According to the above-described underpants-shaped absorbent article, the stretching/contracting force of the elastic member is more likely to transmit to the hydrophobic nonwoven fabric and the hydrophilic nonwoven fabric. Further, the action of an oil agent contained in the adhesive increases the hydrophilicity at the joining portion, making the moisture be more smoothly transferred.

In such an underpants-shaped absorbent article, it is desirable that the waist member has a front waist member and a back waist member, that the waist member has a pair of side joining portions that join the front waist member and the back waist member in two lateral end portions, and that the at least partial region of the waist member is provided in any portion between the pair of side joining portions in the lateral direction.

According to the above-described underpants-shaped absorbent article, the "region between the pair of side joining portions" is applied to the waist region in which the wearer is likely to sweat and stuffiness is likely to occur while the absorbent article is put on. Therefore, moisture is likely to be absorbed in the waist region of the wearer and evaporated to the atmosphere, and this can make the wearer less likely to feel discomfort.

In such an underpants-shaped absorbent article, it is desirable that the at least partial region of the waist member is provided in the back waist member.

According to the above-described underpants-shaped absorbent article, while the absorbent article is put on, in the back waist region where the wearer is likely to sweat and stuffiness is likely to occur, moisture is likely to be absorbed and evaporated to the atmosphere. This can make the wearer less likely to feel discomfort.

In such an underpants-shaped absorbent article, it is desirable that at least a part of the hydrophilic nonwoven fabric is arranged farthest on a non-skin side of the waist member.

According to the above-described underpants-shaped absorbent article, the hydrophilic nonwoven fabric is arranged on the outermost layer of the waist member, and it increases the area of the interface between the hydrophilic nonwoven fabric and the atmosphere, making it likely to evaporate the moisture retained in the hydrophilic nonwoven fabric into the atmosphere. Therefore, the moisture can be efficiently evaporated.

In such an underpants-shaped absorbent article, it is desirable that the waist member has a portion having a two-layer structure in which one layer of the hydrophilic nonwoven fabric and one layer of the hydrophobic nonwoven fabric are overlaid.

According to the above-described underpants-shaped absorbent article, not providing extra sheet member in the waist member makes it less likely to inhibit a function of absorbing moisture from the wearer's skin through the hydrophobic nonwoven fabric and evaporating the absorbed moisture from the hydrophilic nonwoven fabric to the atmosphere, and this makes it possible to efficiently evaporate the moisture. Further, with the above-described configuration, the hydrophilic nonwoven fabric is arranged across the entire region of the waist member in the lengthwise direction (from the upper end portion to the lower end portion). This make the moisture possible to be absorbed and evaporated widely in a region where sweating is likely to occur, such as the wearer's buttocks, and this can make the wearer further less likely to feel discomfort.

In such an underpants-shaped absorbent article, an average fiber density of the hydrophobic nonwoven fabric is larger than an average fiber density of the hydrophilic nonwoven fabric.

According to the underpants-shaped absorbent article, the higher the fiber density of the hydrophobic nonwoven fabric, the more likely a capillary phenomenon is to occur. Therefore, when the fiber density of the hydrophobic nonwoven fabric is made larger than the fiber density of the hydrophilic nonwoven fabric, the moisture absorption efficiency of the hydrophobic nonwoven fabric is increased, and this can make the wearer further less likely to feel discomfort.

In such an underpants-shaped absorbent article, it is desirable that the hydrophobic nonwoven fabric has a plurality of compressed portions in which the hydrophobic nonwoven fabric is compressed in a thickness direction.

According to the above-described underpants-shaped absorbent article, in the portion in which the compressed portions are provided, the fiber density of the hydrophobic nonwoven fabric is locally increased, and the capillary phenomenon is more likely to occur. Accordingly, the moisture absorption efficiency of the hydrophobic nonwoven fabric is increased, and this can make the wearer further less likely to feel discomfort. Further, by providing the plurality of compressed portions in a dispersed manner, the stiffness of the nonwoven fabric is prevented from being excessively high, making it likely to maintain good texture.

In such an underpants-shaped absorbent article, it is desirable that the hydrophilic nonwoven fabric has a plurality of hole portions that penetrate the hydrophilic nonwoven fabric in a thickness direction.

According to the above-described underpants-shaped absorbent article, the side wall portion of each of the hole portions can also serve as an interface with the atmosphere, and therefore the contact area between the atmosphere and the non-skin-side sheet is likely to increase compared with the case where the hole portions are not provided. Accordingly, moisture is also evaporated from the side wall portions of the hole portions, making it possible to increase the moisture evaporation rate of the hydrophilic nonwoven fabric. This can make the wearer less likely to feel discomfort.

In such an underpants-shaped absorbent article, it is desirable that an average thickness of the hydrophilic nonwoven fabric is larger than an average thickness of the hydrophobic nonwoven fabric.

According to the above-described underpants-shaped absorbent article, the thicker the hydrophilic nonwoven fabric, the deeper the depth of the hole portion in the thickness direction, and the larger the area of the side wall portion of the hole portion. Accordingly, the area of the interface between the hydrophilic nonwoven fabric and the atmosphere is increased, and the amount of moisture evaporated from the hydrophilic nonwoven fabric is increased. Therefore, the moisture evaporation rate of the hydrophilic nonwoven fabric is increased, and this can make the wearer less likely to feel discomfort.

In such an underpants-shaped absorbent article, it is desirable that a skin-side surface of the hydrophilic nonwoven fabric has a protruding portion in which an outer edge portion of the hole portion protrudes toward a skin side.

According to the above-described underpants-shaped absorbent article, the hydrophilic nonwoven fabric and the hydrophobic nonwoven fabric are likely to come into contact with each other via the protruding portion, enabling to make it likely to generate an operation of transferring the moisture absorbed by the hydrophobic nonwoven fabric to the hydrophilic nonwoven fabric. Accordingly, moisture is less likely to be retained in the hydrophobic nonwoven fabric which is in contact with the wearer's skin, and this can make the wearer less likely to feel discomfort.

In an underpants-shaped absorbent article according to the present invention, it is desirable that the underpants-shaped absorbent article has a portion where the hole portion and the elastic member overlap each other.

According to the above-described underpants-shaped absorbent article, the hole portion is provided at a position which is likely to be affected by stretching/contracting of the elastic member, and therefore the evaporation of moisture from the side wall portion of the hole portion to the atmosphere can be promoted at the time of stretching/contracting of the elastic member. This can make the wearer less likely to feel discomfort.

In an underpants-shaped absorbent article according to the present invention, it is desirable that the plurality of hole portions are provided between two elastic members that are arranged adjacent to each other in the vertical direction.

According to the above-described underpants-shaped absorbent article, the area of the interface between the hydrophilic nonwoven fabric and the atmosphere is increased by providing the plurality of hole portions in a region between the elastic members in the vertical direction. This can make it likely to efficiently evaporate the moisture even in a region which is less likely to be affected by the stretching/contraction of the elastic members. This can make the wearer less likely to feel discomfort.

In an underpants-shaped absorbent article according to the present invention, it is desirable that the hydrophobic nonwoven fabric does not have a through hole that penetrates the hydrophobic nonwoven fabric in the thickness direction.

According to the above-described underpants-shaped absorbent article, the through hole is not provided in the hydrophobic nonwoven fabric, and therefore the area of the portion in which the hydrophobic nonwoven fabric and the wearer's skin come into contact with each other is not decreased, making the hydrophobic nonwoven fabric likely to efficiently absorb moisture. This can make the wearer less likely to feel discomfort.

In an underpants-shaped absorbent article according to the present invention, it is desirable that an average basis weight of the hydrophilic nonwoven fabric is larger than an average basis weight of the hydrophobic nonwoven fabric.

According to the above-described underpants-shaped absorbent article, the basis weight of the hydrophilic nonwoven fabric is made larger than the basis weight of the hydrophobic nonwoven fabric, and this makes it possible to increase the water retaining capacity in the hydrophilic nonwoven fabric. Accordingly, the moisture is likely to be transferred from the hydrophobic nonwoven fabric to the hydrophilic nonwoven fabric. Further, when the moisture in the hydrophobic nonwoven fabric is transferred to the hydrophilic nonwoven fabric, the hydrophobic nonwoven fabric can newly absorb moisture from the wearer's skin. This can make the wearer less likely to feel discomfort.

### First Embodiment

The following describes an underpants-shaped absorbent article according to the present invention by way of example of an underpants-shaped disposable diaper (hereinafter, also referred to as a "diaper 1"). However, the underpants-shaped absorbent article according to the present invention includes an underpants-shaped napkin, and other types of underpants-shaped absorbent article.

### Configuration of Diaper 1

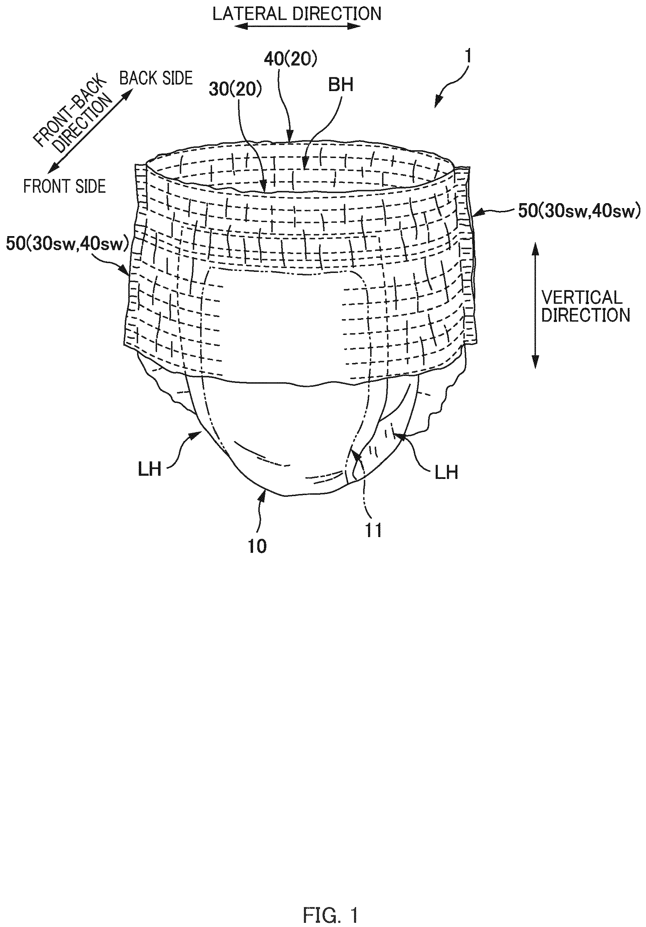

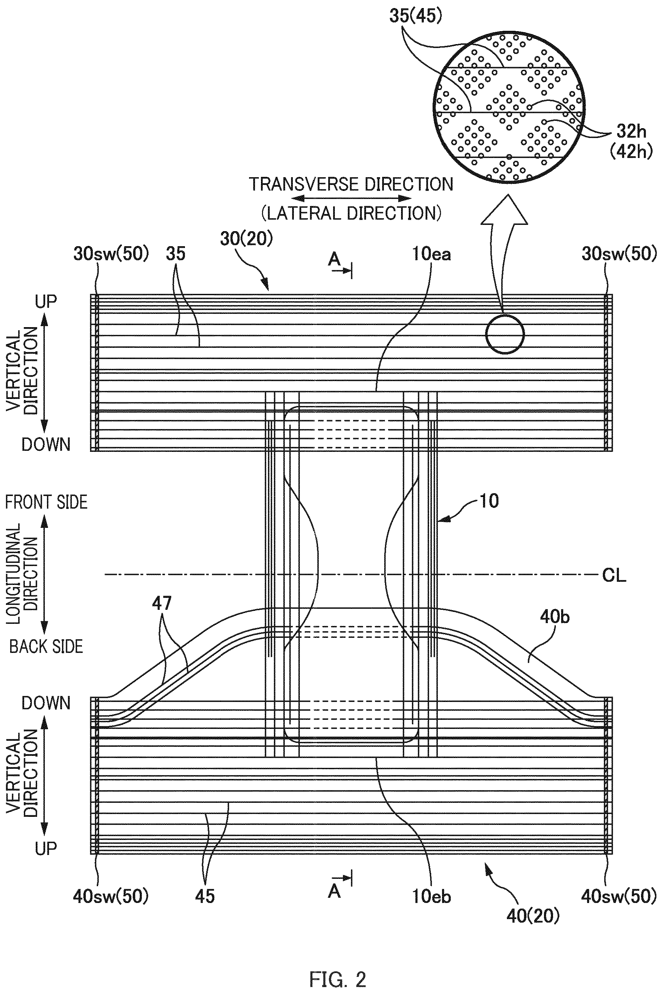

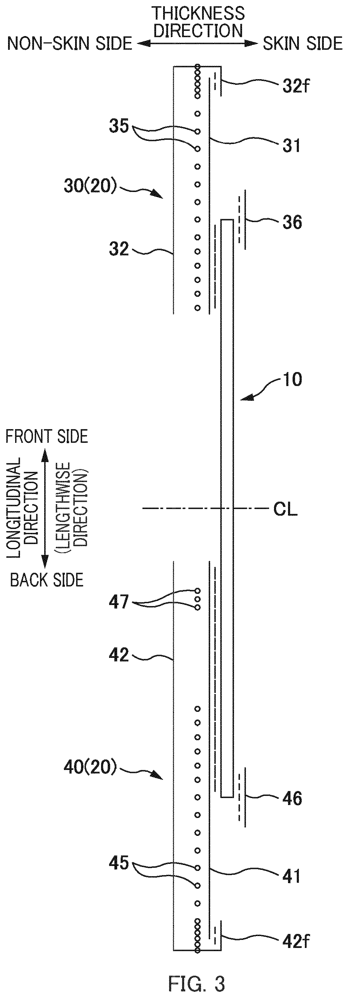

FIG. 1 is a schematic perspective view of a diaper 1. FIG. 2 is a plan view of the diaper 1 in an unfolded and stretched state. FIG. 3 is a schematic cross-sectional view taken along a line A-A in FIG. 2. It should be noted that the "stretched state" of the diaper 1 refers to a state where the entire diaper 1 (the entire product) is stretched without being wrinkled, specifically, a state where the diaper is stretched such that the dimensions of constituent members of the diaper 1 (e.g., an absorbent main body 10, a waist member 20, or the like to be described later) match or are close to the dimensions of the members on their own.

In an underpants-shaped state shown in FIG. 1, the diaper 1 has a vertical direction, a lateral direction, and a front-back direction that intersect with each other and has a waist opening BH and a pair of leg openings LH and LH. The upper side in the vertical direction corresponds to the waist opening BH side, and the lower side corresponds to the crotch side. In addition, the front side in the front-back direction corresponds to the wearer's front side, and the back side corresponds to the wearer's back side. Further, in an unfolded state in FIG. 2, the diaper 1 has a longitudinal direction and a transverse direction that intersect with each other. The longitudinal direction is a direction extending along the vertical direction in FIG. 1 and corresponds to the lengthwise direction of the absorbent main body 10. The transverse direction is a direction extending along the lateral direction in FIG. 1. In addition, as shown in FIG. 3, a direction in which constituent members of the diaper 1 are overlaid is referred to as a thickness direction. In the thickness direction, the side that comes into contact with the wearer's skin is defined as the skin side, and the side opposite to the skin side is defined as the non-skin side.

The diaper 1 has a liquid-absorbent absorbent main body 10, and a waist member 20 that is arranged on the non-skin side of the absorbent main body 10. The waist member 20 has a front waist portion 30 corresponding to the front panel of the diaper 1 and a back waist portion 40 corresponding to the back panel of the diaper 1. That is, the diaper 1 of the first embodiment is a so-called three-piece type underpants-shaped diaper including: as a first component, an absorbent main body 10 that is applied to the wearer's crotch portion and absorbs excrement such as urine or the like; as a second component, a front waist portion 30 that covers the wearer's stomach side portion; and as a third component, a back waist portion 40 that covers the wearer's back side portion.

In an unfolded state in FIG. 2, in a state where the front waist portion 30 and the back waist portion 40 are arranged side by side in the longitudinal direction with a space with respect to each other, lengthwise (longitudinal) end portions 10ea and 10eb of the absorbent main body 10 are respectively joined and fixed to the skin side of the nearest waist portions 30 and 40, while the absorbent main body 10 is spanned between the front waist portion 30 and the back waist portion 40. The external shape thereof forms a substantially H shape in a plan view. Then, from this state, the absorbent main body 10 is folded one time at a fold position, which is the lengthwise central position CL. In this folded state, the front waist portion 30 and the back waist portion 40 that face each other are joined and connected to each other at two lateral side portions 30sw and 40sw to form a pair of side joining portions 50 and 50. That is, the front waist portion 30 and the back waist portion 40 are shaped into an annular shape by the pair of side joining portions 50 and 50. Accordingly, the diaper 1 is in an underpants-shaped state in which the waist opening BH and the pair of leg openings LH and LH are formed as shown in FIG. 1. It should be noted that the side joining portion 50 is formed by commonly-known joining means such as welding or adhesive.

### Absorbent Main body 10

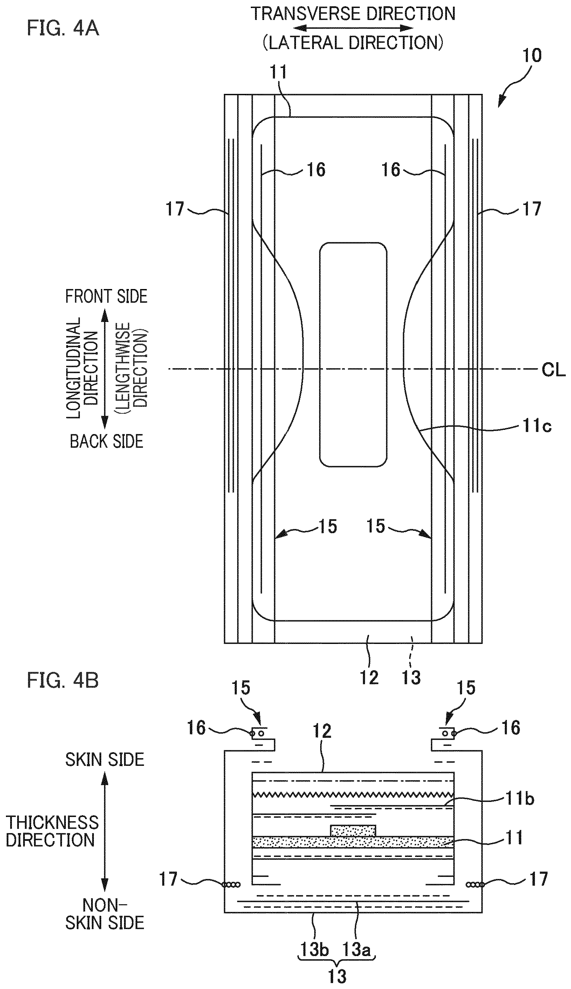

FIG. 4A is a plan view of the absorbent main body 10, and FIG. 4B is a schematic cross-sectional view of the absorbent main body 10.

The absorbent main body 10 has an absorbent core 11 that absorbs excreted fluids, a top sheet 12 that is arranged on the skin side in the thickness direction with respect to the absorbent core 11, and a back sheet 13 that is arranged on the non-skin side with respect to the absorbent core 11. However, the absorbent main body 10 may include other sheet members. For example, a second sheet (not shown) may be provided between the top sheet 12 and the absorbent core 11 in the thickness direction.

The absorbent core 11 is a member that absorbs and holds excreted fluid such as urine, and is formed of, for example, a liquid absorbent fiber (e.g., a pulp fiber) containing a superabsorbent polymer (SAP). It should be noted that the outer circumferential surface of the absorbent core 11 may be covered with a liquid-permeable sheet member (core-wrapping sheet 11b) such as tissue paper or nonwoven fabric. The absorbent core 11 of the present embodiment has a narrow portion 11c, which has a narrow width in the lateral direction, between a front end and a back end in the lengthwise direction, and has a substantially hourglass shape in a plan view as shown in FIG. 4A. This narrow portion 11c is a portion sandwiched between the wearer's two legs while the diaper 1 is put on, and the lateral length thereof is small (the width is narrow), making the absorbent core 11 easier to fit to the wearer's crotch. Further, in the present embodiment, as shown in FIG. 4B, the absorbent core 11 is in a state where two layers of the absorbent core are overlaid on each other in the thickness direction, but the shape of the absorbent core 11 is not limited thereto.

The top sheet 12 is a liquid-permeable sheet, and, for example, a hydrophilic air-through nonwoven fabric, spunbond nonwoven fabric, or the like may be used. In the present embodiment, as shown in FIG. 4B, two lateral side portions are folded back toward the non-skin side so as to enclose the absorbent core 11.

The back sheet 13 has a two-layer structure including a liquid-impermeable sheet 13a and a hydrophobic exterior sheet 13b arranged on the non-skin side of the liquid-impermeable sheet 13a. For example, as the liquid-impermeable sheet 13a, a resin film or the like is used, and as the exterior sheet 13b, a flexible nonwoven fabric sheet is used.

On two lateral side portions of the absorbent main body 10, a pair of the leak-proof wall portions 15 are respectively provided along the longitudinal direction (the lengthwise direction of the absorbent main body 10). In the present embodiment, the leak-proof wall portion 15 is formed by the above-described exterior sheet 13b. Specifically, in the lateral direction (transverse direction), a part of the exterior sheet 13b is folded toward the skin side at a plurality of locations as shown in FIG. 4B, while extending outward with respect to two end portions of the absorbent core 11, to form the pair of leak-proof wall portions 15. To the skin-side end portion (leading end portion) of each leak-proof wall portion 15, leak-proof-wall elastic members 16 such as elastic strings are attached in a state of being stretched along the longitudinal direction (the lengthwise direction of the absorbent main body 10). While the diaper 1 is put on, the leak-proof wall portions 15 rise toward the wearer's skin side and fit to the wearer's crotch portion due to the stretchability developed by the leak-proof-wall elastic members 16.

Further, to two lateral side portions of the absorbent main body 10, leg elastic members 17 such as elastic strings are attached in a state of being stretched along the longitudinal direction (the lengthwise direction of the absorbent main body 10). While the diaper 1 is put on, the two side portions of the absorbent main body 10 contract and becomes more likely to fit around the wearer's legs due to the stretchability developed by the leg elastic members 17.

### Front Waist Portion 30

As shown in FIG. 3, the front waist portion 30 includes: a skin-side sheet 31 that is arranged farthest on the skin side in the thickness direction; a non-skin-side sheet 32 that is overlaid so as to be adjacent to the non-skin side of the skin-side sheet 31; and waist elastic members 35 that are provided between the skin-side sheet 31 and the non-skin-side sheet 32 in the thickness direction. The front waist portion 30 basically has a two-layer structure constituted by the skin-side sheet 31 and the non-skin-side sheet 32, but may partially have a configuration of three or more layers including a skin surface sheet 36 or the like described below.

The skin-side sheet 31 and the non-skin-side sheet 32 are sheet members having a rectangular shape in a plan view as shown in FIG. 2, and are formed of, for example, an SMS nonwoven fabric or the like. It should be noted that in the diaper 1, the sheet member (nonwoven fabric sheet) that constitutes the non-skin-side sheet 32 has higher hydrophilicity than the sheet member (nonwoven fabric sheet) that constitutes the skin-side sheet 31. That is, the skin-side sheet 31 is formed of a nonwoven fabric sheet having low hydrophilicity (hereinafter, also referred to as a "hydrophobic nonwoven fabric"), and the non-skin-side sheet 32 is formed of a nonwoven fabric sheet having higher hydrophilicity than the skin-side sheet 31 (hereinafter, also referred to as a "hydrophilic nonwoven fabric"). Details of the hydrophilicity of each of the sheets 31 and 32 and the function based on the difference in hydrophilicity will be described later.

Further, on the surface of the non-skin-side sheet 32, the plurality of hole portions 32h as shown in a partially enlarged view of FIG. 2 are provided. The hole portions 32h are each a through hole that penetrates the non-skin-side sheet 32 in the thickness direction. Providing the hole portions 32h makes it possible to enhance the breathability of the front waist portion 30. Further, arranging the hole portions 32h visibly on the non-skin surface side of the front waist portion 30 makes the user more likely to invoke that the front waist portion 30 has good breathability. Each of the hole portions 32h may have, for example, a circular shape having a diameter of approximately 1 mm, but the shape and arrangement (number and pattern) of the hole portions 32h can be appropriately changed. It should be noted that, in the diaper 1, the through hole corresponding to the hole portion 32h is not provided in the skin-side sheet 31.

The front waist portion 30 of the present embodiment has a folded-back portion 32f in which the upper end portion (the front end portion in the longitudinal direction) of the non-skin-side sheet 32 is folded back from the non-skin side to the skin side and from the front side to the back side in the longitudinal direction. By covering a part (upper end portion) of the skin-side sheet 31 with the folded-back portion 32f, the upper end edge of the skin-side sheet 31 is prevented from biting into the wearer's skin. However, the folded-back portion 32f need not have to be necessarily provided.

Between the skin-side sheet 31 and the non-skin-side sheet 32, a plurality of waist elastic members 35 are arranged side by side in the vertical direction, and are attached in a state of being stretched in the lateral direction. The front waist portion 30 fits around the wearer's front waist due to the stretchability developed by the waist elastic member 35.

The waist elastic members 35 can be attached using an adhesive such as a hot-melt adhesive. For example, it is possible to attach the waist elastic members 35 by applying a hot-melt adhesive to each of them, stretching the waist elastic member 35 at a predetermined stretch factor, and sandwiching the waist elastic member 35 between the skin-side sheet 31 and the non-skin-side sheet 32. That is, the skin-side sheet 31 and the non-skin-side sheet 32 are joined using the adhesive with the waist elastic members 35 interposed therebetween. Further, the waist elastic members 35 may be attached by applying an adhesive to the skin-side sheet 31 side and the non-skin-side sheet 32 side, or the waist elastic member 35 may be attached by welding means in which a later-described welding portion 60 is used.

Further, the front waist portion 30 may have the skin surface sheet 36. As shown in FIG. 3, the skin surface sheet 36 is a sheet member arranged so as to cover the upper end portion (the front end portion in the longitudinal direction) of the absorbent main body 10 from the skin side, and functions as a cover sheet. Accordingly, the upper end edge of the absorbent main body 10 is prevented from biting into the wearer's skin while the diaper 1 is put on. The skin surface sheet 36 is formed of, for example, an SMS nonwoven fabric sheet or the like. It should be noted that the skin surface sheet 36 need not be necessarily provided.

In the front waist portion 30 of the present embodiment, in the case where a sheet member is provided on the skin side with respect to the skin-side sheet 31 or on the non-skin side with respect to the non-skin-side sheet 32, these sheets are arranged so as to cover only a part of the skin-side sheet 31 and the non-skin-side sheet 32. For example, the skin surface sheet 36 in FIG. 3 is provided so as to cover only a part of the skin-side sheet 31, and at least a part of the skin-side sheet 31 is in a state of being exposed to the wearer's skin side.

### Back Waist Portion 40

The back waist portion 40 has substantially the same configuration as the front waist portion 30. That is, the back waist portion 40 includes: a skin-side sheet 41 that is arranged farthest on the skin side in the thickness direction; a non-skin-side sheet 42 that is overlaid so as to be adjacent to the non-skin side of the skin-side sheet 41; and waist elastic members 45 that is provided between the skin-side sheet 41 and the non-skin-side sheet 42 in the thickness direction. Further, similar to the front waist portion 30, the back waist portion 40 may have hole portions 42h, a folded-back portion 42f, a skin surface sheet 46, and the like (see FIGS. 2 and 3). The configuration of the members is substantially the same as that of the front waist portion 30, and therefore description thereof is omitted.

On the other hand, the external shape of the back waist portion 40 is different from the external shape of the front waist portion 30. Specifically, as shown in FIG. 2, the back waist portion 40 has a crotch region 40b whose lower portion in the vertical direction with respect to the side joining portion 50 (side portion 40sw) has a substantially trapezoidal shape. In the crotch region 40b, the lateral width is widened from the lower side toward the upper side in the vertical direction, and providing the crotch region 40b allows the back waist portion 40 to cover the wearer's buttocks while the diaper 1 is put on. That is, the crotch region 40b functions as a buttocks cover.

Further, in the crotch region 40b, curved elastic members 47 such as elastic strings as shown in FIG. 2 are provided. The curved elastic members 47 are each attached between the skin-side sheet 41 and the non-skin-side sheet 42 in a state of being stretched along the outer edge of the crotch region 40b. The stretchability developed by the curved elastic members 47 makes the crotch region 40b of the back waist portion 40 likely to fit to the wearer's buttocks while the diaper 1 is put on, and also makes it difficult to be turned up from the buttocks.

### Hydrophilicity of Sheet Member

Here, the hydrophilicity of the sheet member will be described. In the diaper 1, a hydrophobic nonwoven fabric is used as the skin-side sheet 31, and a hydrophilic nonwoven fabric having higher hydrophilicity than the skin-side sheet 31 is used as the non-skin-side sheet 32. The hydrophilic nonwoven fabric of the present embodiment has increased hydrophilicity by undergoing a treatment for attaching a predetermined oil agent to the hydrophobic nonwoven fabric (hydrophilic treatment). As the oil agent used in the hydrophilic treatment, it is possible to use commercially available oil agents having an effect as antistatic agents for fibers, such as anionic oil agents, nonionic oil agents, and blends thereof. These oil agents are put into an oil tank and then subjected to oiling with an oiling roller or the like. As a result, the hydrophilicity of the hydrophobic nonwoven fabric can be enhanced, obtaining the hydrophilic nonwoven fabric. However, the hydrophilic nonwoven fabric may be formed by other methods. For example, a hydrophilic nonwoven fabric may be obtained by manufacturing a nonwoven fabric using highly hydrophilic fibers.

It should be noted that, in the present embodiment, the entire nonwoven fabric that constitutes the non-skin-side sheet 32 is subject to the hydrophilic treatment, and the hydrophilicity of the entirety of the non-skin-side sheet 32 is enhanced. However, a configuration is possible in which the hydrophilicity is enhanced only in a partial region of the non-skin-side sheet 32. For example, the sheet member may have a locally high hydrophilic portion and a locally low hydrophilic portion by undergoing a hydrophilic treatment only on the partial region of the non-skin-side sheet 32.

The hydrophilicity of a sheet member can be evaluated by measuring a contact angle when ion exchange water is brought into contact with the surface of the sheet member. Specifically, in the case where the contact angle between the hydrophilic nonwoven fabric and the ion exchange water is smaller than the contact angle between the hydrophobic nonwoven fabric and the ion exchange water, the hydrophilicity of the hydrophilic nonwoven fabric becomes higher than the hydrophilicity of the hydrophobic nonwoven fabric. For the hydrophilic nonwoven fabric (non-skin-side sheet 32) used in the present embodiment, the contact angle with the ion exchange water is desirably less than 90 degrees, and more desirably 50 degrees or less. On the other hand, for the hydrophobic nonwoven fabric (skin-side sheet 31), the contact angle with ion exchange water is desirably 90 degrees or more, and more desirably 120 degrees or more.

The contact angle can be measured by the following method using, for example, a contact angle meter MCA-J manufactured by Kyowa Interface Science Co., Ltd. First, ion exchange water is dropped (approximately 20 picoliters) onto the surface of the fibers that constitute a sheet member (sheet to be measured), and then immediately the contact angle is measured using the contact angle meter. The measurement is performed at a plurality of places (e.g., five or more places) on the surface of the sheet to be measured, and the average value of these positions is defined as the contact angle. It should be noted that the measurement environment temperature is set to 22°C.

Alternatively, the contact angle may be measured by capturing images of the sheet to be measured onto which ion exchange water is dropped from the cross-sectional direction of the sheet to be measured, analyzing the captured image, and measuring the angle between the ion exchange water droplet and the sheet to be measured.

### Moisture Absorption and Evaporation

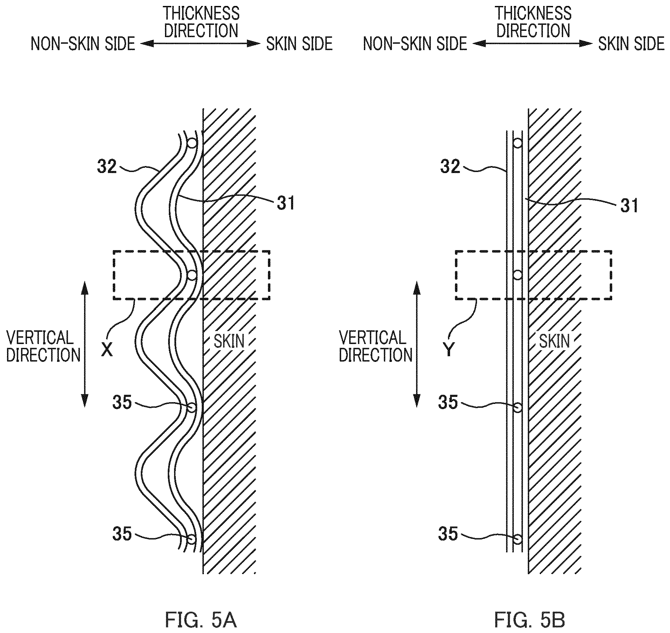

Next, a mechanism of absorbing moisture from the wearer's skin and evaporating the absorbed moisture to the atmosphere in the waist member 20 of the diaper 1 will be described. FIGS. 5A and 5B are diagrams illustrating the cross-sectional shape of the front waist portion 30 while the diaper 1 is put on. FIGS. 5A and 5B schematically show cross sections in the thickness direction and the vertical direction, FIG. 5A shows a state where the waist elastic members 35 contract in the lateral direction, and FIG. 5B shows a state where the waist elastic members 35 stretch in the lateral direction. It should be noted that, in the following, the front waist portion 30 will be described, but the back waist portion 40 is similar to the front waist portion 30.

When the wearer puts on the diaper 1, the waist elastic members 35 provided in the front waist portion 30 contract in the lateral direction, and this makes the front waist portion 30 fit around the wearer's waist, making it less likely to cause positional deviation and the like. At this time, accompanying with contraction of the waist elastic members 35 in the lateral direction, the skin-side sheet 31 and the non-skin-side sheet 32 which are joined with the waist elastic members 35 interposed therebetween also contract in the lateral direction. Therefore, as shown in FIG. 1, a plurality of wrinkles that extend in the vertical direction are formed on the surface of the front waist portion 30. These wrinkles are formed by the sheet member that was in a flat state partially protruding in the thickness direction when the non-skin-side sheet 32 (and the skin-side sheet 31) contracts in the lateral direction. In FIG. 5A, small wrinkles are formed between two waist elastic members 35 and 35 which are adjacent to each other in the vertical direction by the non-skin-side sheet 32 protruding toward the non-skin side in the thickness direction (toward the side that does not face the wearer's skin), and wrinkles that extend in the vertical direction are formed by arranging these small wrinkles side by side in the vertical direction.

From this state, when the wearer moves the body while walking, sitting on or standing up, the contracted waist elastic members 35 are stretched, and in conjunction therewith, it generates a portion in which the skin-side sheet 31 and the non-skin-side sheet 32 are also stretched in the lateral direction. As a result, in the stretched portion, the protrusion of the skin-side sheet 31 and the non-skin-side sheet 32 in the thickness direction is eliminated, and the sheets become flat along the skin as shown in FIG. 5B. In a state where the wearer wears the diaper 1, the contracted state (FIG. 5A) and the stretched state (FIG. 5B) as described above are repeated.

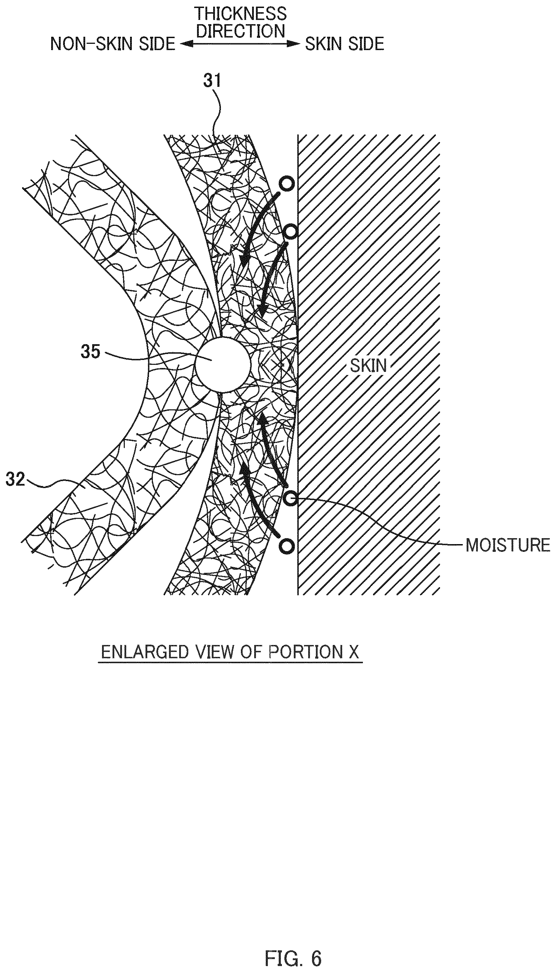

FIG. 6 is a cross-sectional view showing a region X in FIG. 5A in an enlarged manner. In FIG. 6, in the case where the waist elastic member 35 contracts in the lateral direction, the skin-side sheet 31 also contracts in the lateral direction as described above. Therefore, the fibers that constitute the skin-side sheet 31 are compressed in the lateral direction, the inter-fiber distance is narrowed, and the fibers that constitute the skin-side sheet 31 are in a dense state.

In this state, when the skin-side sheet 31 (hydrophobic sheet) is pressed against the wearer's skin side, moisture such as sweat adhering to the wearer's skin is more likely to be absorbed by the skin-side sheet 31. This is because, the fibers that constitute the skin-side sheet 31 are dense, and this makes the moisture is likely to be drawn between the fibers due to the capillary phenomenon. As described above, in the front waist portion 30 of the diaper 1, the skin-side sheet 31 formed of the hydrophobic nonwoven fabric is contracted by the waist elastic member 35, and this can make the moisture likely to be absorbed from the wearer's skin.

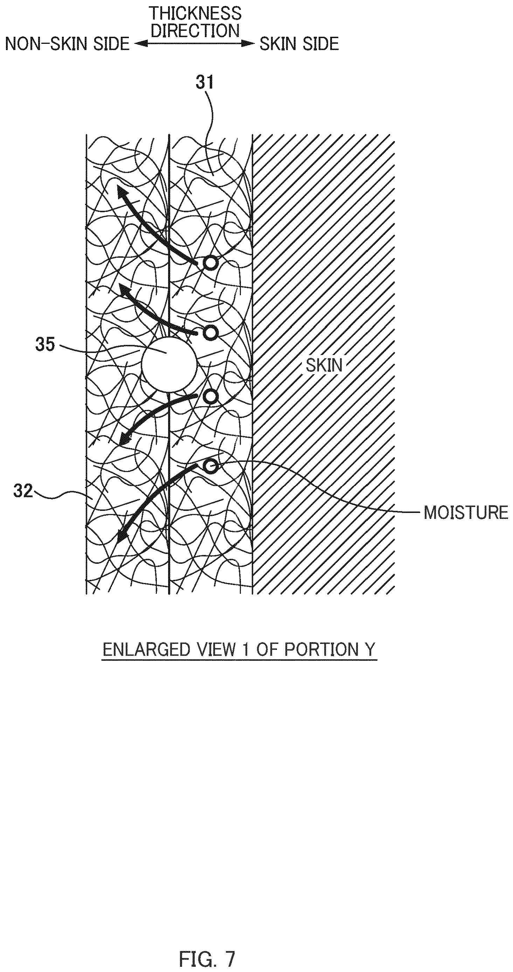

FIG. 7 is a cross-sectional view showing a region Y in FIG. 5B in an enlarged manner. When the waist elastic member 35 is stretched, the skin-side sheet 31 and the non-skin-side sheet 32 both are stretched and become flat, and therefore the non-skin-side surface of the skin-side sheet 31 and the skin-side surface of the non-skin-side sheet 32 become overlaid on each other and more likely to come into surface contact with each other. That is, compared with the case of FIG. 6, the area of the portion where the skin-side sheet 31 and the non-skin-side sheet 32 are in contact with each other is increased. Then, the moisture absorbed by the skin-side sheet 31 is diffused in the non-skin-side sheet 32 while moving from the skin-side sheet 31 side to the non-skin-side sheet 32 side through the contact portion interposed therebetween. This is because, in the present embodiment, the hydrophilicity of the non-skin-side sheet 32 formed of the hydrophilic nonwoven fabric is higher than the hydrophilicity of the skin-side sheet 31 formed of the hydrophobic nonwoven fabric, and this makes the moisture likely to transfer, being diffused to the highly hydrophilic sheet. Accordingly, moisture is less likely to remain in the skin-side sheet 31 and less likely to come into contact with the wearer's skin.

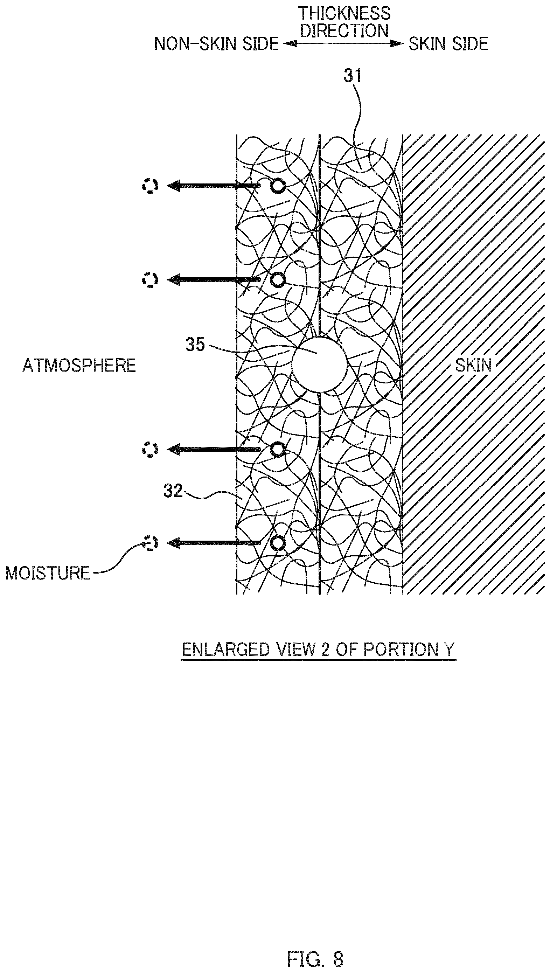

FIG. 8 is an enlarged cross-sectional view of a region Y in FIG. 5B, and shows a state after a predetermined time has elapsed from the state of FIG. 7. The moisture that has been transferred from the skin-side sheet 31 to the non-skin-side sheet 32 is diffused into the highly hydrophilic non-skin-side sheet 32 and is evaporated to the outside (non-skin side) from the non-skin-side surface of the non-skin-side sheet 32 (in FIG. 8, the interface between the non-skin-side sheet 32 and the atmosphere). In the present embodiment, accompanying with the stretching of the waist elastic member 35, the non-skin-side sheet 32 itself is stretched, and this makes it likely to promote the diffusion of moisture in the non-skin-side sheet 32, making it more likely to cause moisture evaporation in a wide range of the non-skin-side sheet 32. Then, the moisture retained in the non-skin-side sheet 32 is evaporated to the non-skin side, making it likely to newly transfer moisture from the skin-side sheet 31 to the non-skin-side sheet 32.

In this manner, in the front waist portion 30 of the diaper 1, moisture can be absorbed from the wearer's skin, and the absorbed moisture can be evaporated to the non-skin side of the non-skin-side sheet 32 (hydrophilic nonwoven fabric). Then, when the wearer moves the body in a state where the diaper 1 is put on, the waist elastic member 35 stretches and contracts, and the movement of moisture described in FIGS. 6 to 8 is repeated. Accordingly, moisture is efficiently absorbed from the wearer's skin and released to the non-skin side, and this can make the wearer less likely to feel discomfort.

It should be noted that, even when another sheet member is overlaid on the non-skin side of the non-skin-side sheet 32, the above-described effect can be realized. However, in the case where no other sheet member is overlaid on the non-skin side of the non-skin-side sheet 32 (that is, in the case where the non-skin-side surface of the non-skin-side sheet 32 faces the atmosphere), the evaporation efficiency can be further increased. Therefore, it is desirable that the non-skin-side sheet 32 is arranged farthest on the non-skin side in the waist member 20 (front waist portion 30) of the diaper 1.

Further, in the front waist portion 30, the waist elastic members 35 are provided between the skin-side sheet 31 and the non-skin-side sheet 32, and therefore the front waist portion 30 has a configuration making moisture likely to be absorbed from the wearer's skin and to be evaporated into the atmosphere. If the waist elastic members 35 are arranged on the skin side with respect to the skin-side sheet 31, a gap is more likely to be generated between the wearer's skin and the skin-side sheet 31. That is, the contact area between the wearer's skin and the skin-side sheet 31 is decreased, causing a risk that moisture is less likely to be absorbed from the wearer's skin. Further, if the waist elastic members 35 are arranged on the non-skin side with respect to the non-skin-side sheet 32, there is a risk that the front waist elastic members 35 prevents the evaporation of moisture from the non-skin side surface of the non-skin-side sheet 32 to the atmosphere, making the moisture less likely to be evaporated. In contrast, in the present embodiment, since the waist elastic members 35 are provided between the skin-side sheet 31 and the non-skin-side sheet 32, and therefore the movement of moisture is less likely to be inhibited, and the moisture can be efficiently absorbed and evaporated.

Further, in the diaper 1, the movement of moisture as described above is likely to be caused by the stretching and contraction of the waist elastic members 35. Therefore, it is desirable that a portion in which the skin-side sheet 31 (hydrophobic nonwoven fabric) and the non-skin-side sheet 32 (hydrophilic nonwoven fabric) are joined to each other is provided around each waist elastic member 35. According to the above-described configuration, the stretching and contraction of the waist elastic members 35 has a large influence, and this can enhance the moisture evaporation effect. That is, since the skin-side sheet 31 and the non-skin-side sheet 32 are also easily stretched and contracted according to the stretching and contraction of the waist elastic member 35, the movement of moisture is likely to be promoted. Further, since the sheets are in close contact with each other in the joining portion, moisture is more smoothly transferred from the skin-side sheet 31 having low hydrophilicity to the non-skin-side sheet 32 having high hydrophilicity.

Further, in the front waist portion 30 (waist member 20) of the diaper 1, by using an adhesive such as a hot-melt adhesive, the skin-side sheet 31 (hydrophobic nonwoven fabric) and the non-skin-side sheet 32 (hydrophilic nonwoven fabric) are joined to each other via the waist elastic members 35. Therefore, the stretching/contracting force of the waist elastic members 35 is directly transmitted to the skin-side sheet 31 and the non-skin-side sheet 32, making it possible to further enhance the moisture evaporation effect. Further, the action of the oil agent contained in the adhesive increases the hydrophilicity at the joining portion in which the sheets are joined, making the moisture be more smoothly transferred.

Further, in the diaper 1, in a region between the pair of side joining portions 50 and 50 in the lateral direction, the waist elastic members 35 and 45 are provided between the skin-side sheets 31 and 41 (hydrophobic nonwoven fabric) and the non-skin-side sheets 32 and 42 (hydrophilic nonwoven fabric). Therefore, the diaper 1 has a configuration in which moisture is likely to be absorbed from the wearer's skin and the absorbed moisture is likely to be released to the atmosphere in at least any region between the pair of side joining portions 50 and 50 in the waist member 20. This region is a region positioned around the wearer's waist while the diaper 1 is put on, and is a region in which the wearer is likely to sweat and stuffiness is likely to occur. In the present embodiment, it can make moisture likely to be absorbed and evaporated to the atmosphere in the waist region, and therefore while putting on the diaper 1, stuffiness in the waist region is suppressed, enabling to make the wearer less likely to feel discomfort.

In particular, while the wearer puts on the underpants-shaped diaper, the wearer is more likely to sweat on the back side of the waist region than on the front side, making stuffiness likely to occur on the back-side. In contrast, in the diaper 1, in the back waist portion 40, in a region between the pair of side joining portions 50 and 50 in the lateral direction, the waist elastic members 45 are provided between the skin-side sheet 41 (hydrophobic nonwoven fabric) and the non-skin-side sheet 42 (hydrophilic nonwoven fabric). This can make moisture such as sweat more likely to efficiently absorbed and evaporated to the atmosphere in the waist region, and stuffiness is suppressed in the back waist region, and this can make the wearer less likely to feel discomfort.

Further, in the diaper 1 of the present embodiment, the non-skin-side sheets 32 and 42 formed of the hydrophilic nonwoven fabric are arranged farthest on the non-skin side in the thickness direction of the waist member 20 (30 and 40) (see FIG. 3). That is, the hydrophilic nonwoven fabric is arranged in at least a part of the outermost layer of the waist member 20. Accordingly, at least a part of the hydrophilic nonwoven fabric serves as an interface with the atmosphere, and the moisture retained in the hydrophilic nonwoven fabric is likely to be evaporated into the atmosphere. In the case where the hydrophobic sheet member is provided overlaid on the non-skin side with respect to the hydrophilic nonwoven fabric, in the overlaid portion, there is a risk that the evaporation of moisture from the hydrophilic nonwoven fabric to the atmosphere is inhibited, making it more likely to stay in the hydrophilic nonwoven fabric. In contrast, in the present embodiment, since there is a portion in which no other member is interposed between the hydrophilic nonwoven fabric and the atmosphere, moisture can be efficiently evaporated. It should be noted that, even when another member (e.g., a post handling tape for performing a post handling operation on the diaper 1) is provided on a part of the non-skin-side surface of the non-skin-side sheets 32 and 42 (hydrophilic nonwoven fabric), the above-described effect can be obtained in a region in which the other member is not provided.

Further, it is preferable that the waist member 20 (30, 40) has a two-layer structure in which two sheet members are overlaid in the thickness direction, namely a single skin-side sheet 31 (41) formed of hydrophobic nonwoven fabric and a single non-skin-side sheet 32 (42) formed of hydrophilic nonwoven fabric. Not providing extra sheet member in the waist member 20 makes it less likely to inhibit the absorption and evaporation of moisture mentioned above, and this makes it possible to more efficiently evaporate the moisture. The waist member 20 of the present embodiment partially has a three-layer structure including a folded-back portion 32f or the like shown in FIG. 3. However, the two-layer structure in which two sheet members, that is, the skin-side sheets 31 and 41 and the non-skin-side sheets 32 and 42, are overlaid on each other in the most region of the waist member 20, and the moisture can be efficiently evaporated.

Further, in such a structure, the hydrophilic nonwoven fabric is arranged across the entirety of the waist member 20 (30 and 40) throughout the longitudinal direction (the vertical direction of the diaper 1), and therefore the moisture absorption and evaporation effect can be exhibited across from the upper end portion to the lower end portion of the waist member 20. For example, moisture such as sweat can be likely to be discharged to the outside of the diaper 1 in a wide region from the back to the buttocks on the wearer's back side (back waist member 40) while the wearer puts on the diaper 1. Therefore, in the lower end region of the waist portion such as buttocks, where the wearer is likely to sweat, moisture is less likely to come into contact with the skin, and this can make the wearer less likely to feel discomfort.

However, this does not exclude providing another sheet member on the non-skin side with respect to the hydrophilic nonwoven fabric (non-skin-side sheets 32 and 42) in the waist member 20. For example, the skin-side sheet 31 (hydrophobic nonwoven fabric) may be folded back toward the non-skin side and overlaid so as to cover a part of the non-skin-side surface of the non-skin-side sheet 32. Even in such a case, the moisture evaporation effect is maintained in the non-overlaid portion, and this can make the wearer less likely to feel discomfort.

Further, it is desirable that in the waist member 20 of the diaper 1, the average fiber density of the hydrophobic nonwoven fabric (skin-side sheets 31 and 41) is larger than the average fiber density of the hydrophilic nonwoven fabric (non-skin-side sheets 32 and 42). In the present embodiment, when the waist elastic members 35 and 45 contract, the hydrophobic nonwoven fabric absorbs moisture from the wearer's skin by utilizing the capillary phenomenon caused by a reduction in the inter-fiber distance between the fibers that constitute the hydrophobic nonwoven fabric. Therefore, the higher the fiber density of the hydrophobic nonwoven fabric, the more likely the capillary phenomenon is to occur, and this can make moisture more likely to be absorbed. Further, the movement of moisture from the hydrophobic nonwoven fabric to the hydrophilic nonwoven fabric is caused based on a difference in hydrophilicity between the nonwoven fabrics. Therefore, even when the fiber density of the hydrophobic nonwoven fabric is increased, it is less likely to inhibit the function of evaporating the absorbed moisture into the atmosphere. Therefore, when the fiber density of the hydrophobic nonwoven fabric is made larger than the fiber density of the hydrophilic nonwoven fabric, compared with the case where the fiber density of the hydrophilic nonwoven fabric is smaller than the fiber density of the hydrophilic nonwoven fabric, the moisture (sweat) absorption efficiency of the hydrophobic nonwoven fabric is enhanced, enabling to make the wearer further less likely to feel discomfort. It should be noted that the average fiber density of the nonwoven fabric is calculated as follow: a sheet piece of a predetermined size is cut out from the to-be-measured nonwoven fabric; the weight and the later-described average thickness of the sheet piece is measured; and the weight of the sheet piece is divided by the product of the area and the average thickness of the sheet piece (the volume of the sheet piece).

As a method for increasing the fiber density of the hydrophobic nonwoven fabric (skin-side sheets 31 and 41), it is possible to use, for example, a method for providing compressed portions obtained by compressing parts of the hydrophobic nonwoven fabric in the thickness direction. Specifically, a plurality of compressed portions may be formed in a dispersed manner by subjecting the surface of the hydrophobic nonwoven fabric to embossing or the like. In the portion in which such compressed portions (embossments) are provided, the fiber density of the hydrophobic nonwoven fabric is locally increased, and the capillary phenomenon is more likely to occur. Further, by providing the fine compressed portions in a dispersed manner, the stiffness of the nonwoven fabric is prevented from being excessively high, making it likely to maintain good texture. Accordingly, the moisture (sweat) absorption efficiency of the hydrophobic nonwoven fabric is enhanced, and this can make the wearer less likely to feel discomfort.

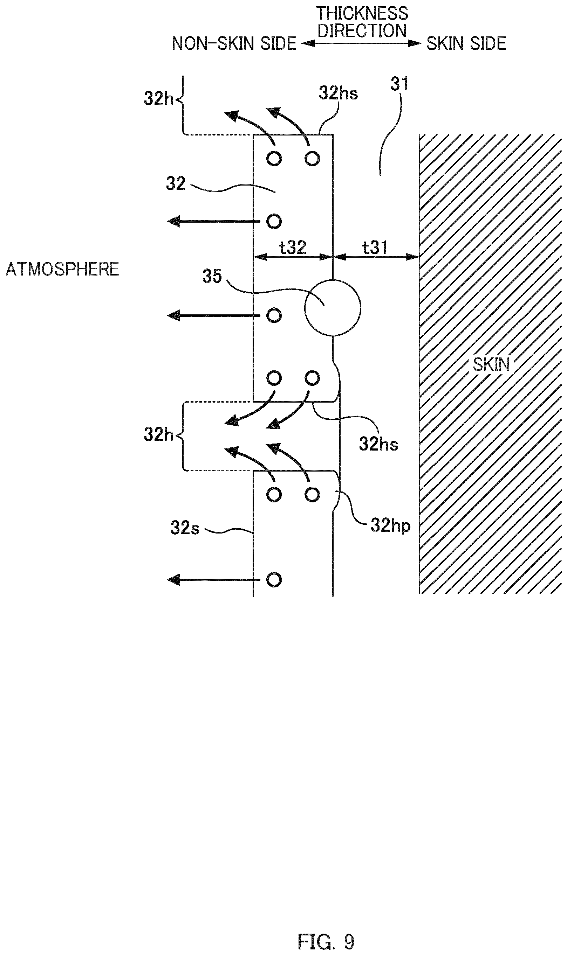

Further, in the waist member 20 of the diaper 1, the plurality of hole portions 32h and 42h that penetrate the hydrophilic nonwoven fabric (non-skin-side sheets 32 and 42) in the thickness direction are provided, and this can make the moisture more likely to be evaporated. FIG. 9 is a diagram corresponding to FIG. 8 and is an explanatory diagram illustrating evaporation of moisture in the case where the hole portions 32h are provided in the non-skin-side sheet 32. As illustrated in FIG. 8, when evaporating moisture from the non-skin-side sheet 32, the moisture is evaporated into the atmosphere from the interface between the non-skin-side sheet 32 and the atmosphere, that is, from the non-skin-side surface of the non-skin-side sheet 32 (represented by 32s in FIG. 9). In contrast, as in FIG. 9, in the case where the hole portions 32h are provided in the non-skin-side sheet 32, a side wall portion 32hs of each of the hole portions 32h also serve as an interface with the atmosphere. That is, compared with the case where the hole portions 32h are not provided, the area of the interface between the atmosphere and the non-skin-side sheet 32 is likely to increase. Therefore, in addition to the non-skin-side surface 32s of the non-skin-side sheet 32, moisture is also evaporated from the side wall portions 32hs of the hole portions 32h, and a larger amount of moisture diffused and retained in the non-skin-side sheet 32 is more likely to be evaporated. Accordingly, the moisture absorption rate of the hydrophilic nonwoven fabric is enhanced, and this can make the wearer further less likely to feel discomfort.

It should be noted that, in the case where the hole portions 32h are provided in the hydrophilic nonwoven fabric (non-skin-side sheet 32), it is desirable that the thickness t32 of the hydrophilic nonwoven fabric (non-skin-side sheet 32) is larger than the thickness t31 of the hydrophobic nonwoven fabric (skin-side sheet 31). As the thickness t32 of the hydrophilic nonwoven fabric (non-skin-side sheet 32) is thicker, it increases the distance of the hole portion 32h in the thickness direction, which penetrates the non-skin-side sheet 32, (that is, the depth of the hole becomes deeper), increasing the area of the side wall portion 32hs of the hole portion 32h. Accordingly, the area of the interface between the hydrophilic nonwoven fabric and the atmosphere is increased, and the amount of moisture evaporated from the side wall portion 32hs is increased. Therefore, the moisture evaporation rate of the hydrophilic nonwoven fabric is increased, and this can make the wearer less likely to feel discomfort. It should be noted that the thickness of the nonwoven fabric is obtained as follow: in a portion of a to-be-measured nonwoven fabric where the hole portions 32h are not provided, a pressure is applied to a target portion at a contact pressure set to 3 gf/cm² using, for example, a dial thickness gauge ID-C1012C manufactured by Mitutoyo Corporation or an equivalent thereof; and then the thickness is measured at any number of places (5 places), obtaining the average value of the thickness. It should be noted that, in the case where the space between the hole portions 32h and 32h which are arranged adjacent to each other (that is, the pitch between the hole portions 32h) are narrower than the size of the measuring piece of the dial thickness gauge, the measuring piece straddles the hole portion 32h, and there is a risk that the thickness of the portion in which hole portions 32h are not provided is not accurately measured. In such a case, it is preferable that the thickness of a portion of the nonwoven fabric where the hole portions 32h are not provided is measured using a non-contact thickness measuring apparatus such as an ultrasonic thickness meter or a laser displacement meter.

Further, on the skin-side surface of the hydrophilic nonwoven fabric (non-skin-side sheet 32), the outer edge portions of the hole portions 32h may have protruding portions 32hp that protrude toward the skin side (see FIG. 9). By providing such protruding portions 32hp, the hydrophilic nonwoven fabric (non-skin-side sheet 32) is more likely to come into contact with the non-skin-side surface of the hydrophobic nonwoven fabric (skin-side sheet 31) via the protruding portions 32hp. This can make it likely to generate an operation of transferring the moisture absorbed by the hydrophobic nonwoven fabric to the hydrophilic nonwoven fabric side (see FIG. 7). Accordingly, moisture is less likely to remain in the hydrophobic nonwoven fabric (skin-side sheet 31) which is in contact with the wearer's skin, and this can make the wearer less likely to feel discomfort.

It should be noted that the protruding portions 32hp can be formed at the time of opening the hole portions 32h in the manufacturing step of the hydrophilic nonwoven fabric (non-skin-side sheet 32). For example, in the case where the hole portion 32h is formed by piercing the non-skin-side sheet 32 with a predetermined pin from the non-skin side of the non-skin-side sheet 32 toward the skin side in the thickness direction, when the pin penetrates the non-skin-side sheet 32, a burr protruding toward the skin side is formed in the skin-side outer edge portion of the hole portion 32h, and the burr become the protruding portion 32hp.

Further, it is desirable that the front waist portion 30 (back waist portion 40) has a portion in which the hole portion 32h (42h) and the waist elastic member 35 (45) overlap each other. In FIG. 2, at least any hole portion 32h of the plurality of hole portions 32h is provided at a position that overlaps the waist elastic member 35. According to the above-described configuration, when the waist elastic members 35 stretch and contract in the lateral direction, the moisture absorbed by the hydrophobic nonwoven fabric (skin-side sheet 31) is transferred to the hydrophilic nonwoven fabric (non-skin-side sheet 32), and an operation of evaporating the moisture from the interface between the hydrophilic nonwoven fabric (non-skin-side sheet 32) and the atmosphere is likely to be promoted. That is, the hole portions 32h are provided at a position which is likely to be affected by the stretching/contracting of the waist elastic member 35, enabling to make the moisture more likely to be evaporated. This can make the wearer less likely to feel discomfort.

Further, it is desirable that a plurality of hole portions 32h are provided between two waist elastic members 35 and 35 which are arranged adjacent to each other in the vertical direction. In FIG. 2, a large number of hole portions 32h are provided between two waist elastic members 35 and 35 which are adjacent to each other in the vertical direction. Accordingly, in the region between the waist elastic members 35 and 35 in the vertical direction, the area of the interface between the hydrophilic nonwoven fabric (non-skin-side sheet 32) and the atmosphere is increased. This can make it likely to efficiently evaporate the moisture even in the region which is less likely to be affected by the stretching/contraction of the waist elastic members 35. This can make the wearer less likely to feel discomfort.

It should be noted that, in the waist member 20, it is desirable that the hydrophobic nonwoven fabric (skin-side sheets 31 and 41) are not provided with open holes (through holes) corresponding to the hole portions 32h. In the case where the through holes are provided in the hydrophobic nonwoven fabric, the contact area between the hydrophobic nonwoven fabric and the wearer's skin is small, and therefore there is a risk that the absorption of moisture based on the capillary phenomenon is less likely to occur. In contrast, in the diaper 1 of the present embodiment, since the through holes penetrating the hydrophobic nonwoven fabric (skin-side sheets 31 and 41) in the thickness direction are not provided, the contact area between the hydrophobic nonwoven fabric and the wearer's skin is not decreased, enabling the hydrophobic nonwoven fabric to efficiently absorb moisture such as sweat. This can make the wearer less likely to feel discomfort.

Further, it is desirable that in the waist member 20, the basis weight of the hydrophilic nonwoven fabric (non-skin-side sheets 32 and 42) is larger than the basis weight of the hydrophobic nonwoven fabric (skin-side sheets 31 and 41). In a general nonwoven fabric, the larger the basis weight, the larger the amount (water retaining capacity) capable of retaining moisture. Therefore, the basis weight of the hydrophilic nonwoven fabric larger is made larger than the basis weight of the hydrophobic nonwoven fabric, and this makes it possible to further increase the water retaining capacity in the hydrophilic nonwoven fabric. Accordingly, the moisture is more likely to be transferred from the hydrophobic nonwoven fabric to the hydrophilic nonwoven fabric (see FIG. 7). Further, when the moisture in the hydrophobic nonwoven fabric is transferred to the hydrophilic nonwoven fabric, the hydrophobic nonwoven fabric can newly absorb moisture from the wearer's skin. This can make the wearer less likely to feel discomfort. It should be noted that the basis weight is obtained as follow: sheet pieces having a predetermined size are cut out at a plurality of places (e.g., ten places) from a sheet member to be measured; and then the weight of each sheet piece is measured using an electronic balance or the like, obtaining the average value of values obtained by dividing the measured weight by the area of the sheet piece.

### Modified Example of Method for Attaching Waist Elastic Members 35 and 45

In the above-described embodiment, the waist elastic members 35 and 45 are attached to the waist member 20 by adhering means using a hot-melt adhesive or the like, but the method for attaching the waist elastic members 35 and 45 is not limited thereto. For example, the waist elastic members 35 and 45 may be attached to the waist member 20 using welding means such as ultrasonic welding. It should be noted that, since ultrasonic welding is a commonly-known technique, the description of ultrasonic welding is omitted in the present specification.

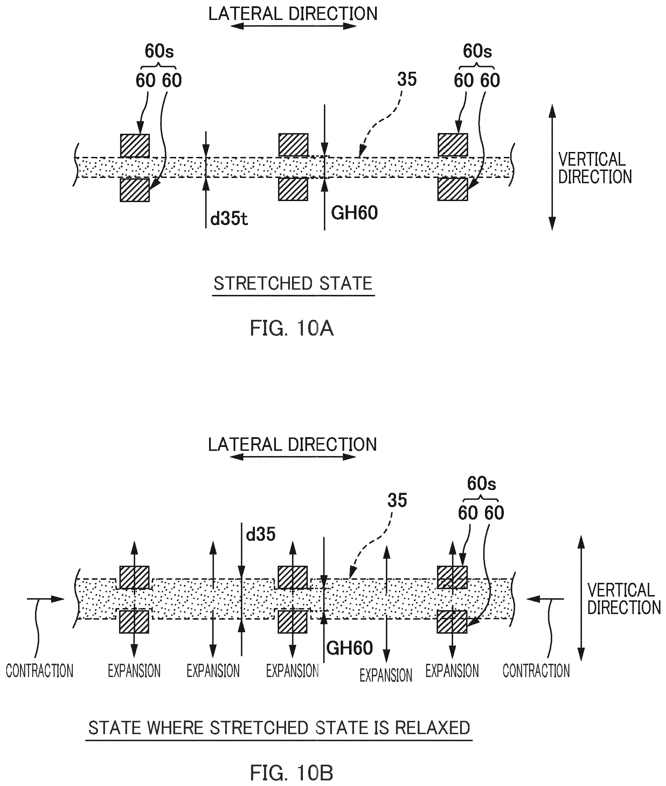

FIGS. 10A and 10B are explanatory diagrams illustrating a method for attaching the waist elastic member 35 to the front waist portion 30 using welding portions 60.

In the present modified example, the waist elastic member 35 is attached to the front waist portion 30 by a plurality of welding portions 60, 60 ..., which are discretely arranged in the lateral direction and the vertical direction. Each of the welding portions 60 is formed into a substantially rectangular shape by ultrasonic welding, and joins the skin-side sheet 31 and the non-skin-side sheet 32 of the front waist portion 30 in the thickness direction. The waist elastic member 35 is attached to the front waist portion 30 by sandwiching the waist elastic member 35 from two vertical sides between welding portion pairs 60s each of which is formed of two welding portions 60 and 60 that are adjacent to each other in the vertical direction.

As shown in FIG. 10A, a pair of welding portions 60 and 60 that constitute the welding portion pair 60s are arranged side by side in the vertical direction with a space GH60. The size of the space GH60 is set to be the same size as or slightly larger than the diameter d35t of the waist elastic member 35 in a state where the waist elastic member 35 is stretched to a predetermined stretch factor (GH60 ≥ d35t). That is, the waist elastic member 35 in the stretched state is arranged between the welding portion pair 60s in the vertical direction.

Next, when the waist elastic member 35 is relaxed from the stretched state, as shown in FIG. 10B, the waist elastic member 35 expands in the vertical direction while contracting in the lateral direction, and the diameter d35 in the natural state becomes larger than the vertical space GH60 of the welding portion pair 60s (d35 > GH60). Accordingly, the waist elastic member 35 is sandwiched between the welding portions 60 and 60 in the vertical direction. As a result, the waist elastic member 35 is attached to the front waist portion 30.

It should be noted that in the diaper 1 in the underpants-shaped state in FIG. 1, the waist elastic members 35 (45) are in a natural state where the waist elastic members 35 (45) are relaxed from the above-described stretched state. Further, in the diaper 1 in the underpants-shaped state, the waist elastic members 35 (45) are joined to the front waist portion 30 (back waist portion 40) by the side joining portions 50 and 50 on two lateral side portions. Therefore, even when the front waist portion 30 (back waist portion 40) is stretched in the lateral direction while the diaper 1 is put on, the waist elastic members 35 (45) are not detached from the waist member 20.

### Inside-Underpants Temperature

Next, the following describes an inside-underpants-temperature measuring experiment of measuring how the internal temperature of the diaper 1 (also referred to as inside-underpants temperature) is changed with time in a state where the diaper 1 is actually put on.

The inside-underpants-temperature measuring experiment was performed as follows using a sweating mannequin which is provided with a temperature sensor at a predetermined position. First, the above-described sweating mannequin is placed in an upright posture in a room maintained at a temperature of 25°C and a humidity of 50%. The sweating mannequin is a large doll capable of intermittently discharging liquid (sweat) from a plurality of sweat pore located on the body surface, and it is possible to use, for example, a sweating mannequin SAM (registered trademark) manufactured by Toyobo Co., Ltd.. This sweating mannequin SAM can reproduce a state similar to the mechanism of sweating from human sweat glands by a "liquid sweat intermittent discharge method" in which liquid is intermittently discharged at a rate of approximately once every several seconds to several tens of seconds from 168 sweat pores provided on the body surface.

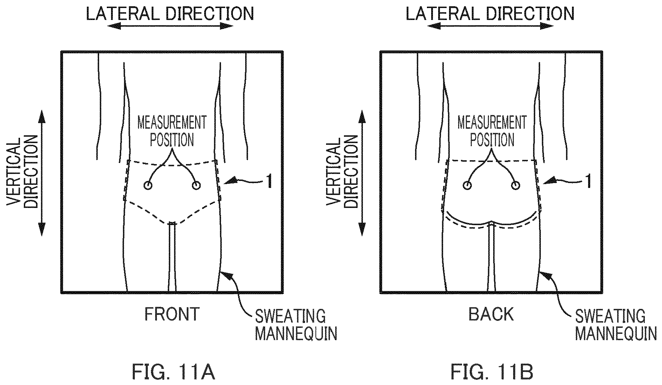

Next, a temperature sensor capable of measuring the temperature is installed at a predetermined position on the body surface of the sweating mannequin. FIG. 11A is an explanatory diagram illustrating the temperature measurement position on the front side of the sweating mannequin, and FIG. 11B is an explanatory diagram illustrating the temperature measurement position on the back side of the sweating mannequin. In the inside-underpants-temperature measuring experiment of the present embodiment, as shown in FIG. 11A, in the front waist portion of the sweating mannequin, the temperature sensors were installed at two front temperature measurement positions that are slightly above the groin and that are symmetrical in the lateral direction with respect to the lateral central position. Further, in the back waist portion of the sweating mannequin, as shown in FIG. 11B, the temperature sensors were installed at two back temperature measurement positions where the protrusion of the buttocks is the largest and that are symmetrical in the lateral direction with respect to the lateral central position.

Next, an underpants-shaped wearing article (e.g., diaper 1) to be tested is put on the sweating mannequin. At this time, the underpants-shaped wearing article is put on so as to cover the temperature measurement positions (corresponding to the predetermined positions) (see FIGS. 11A and 11B). That is, the underpants-shaped wearing article is put on so that the temperature measurement positions are positioned inside the underpants-shaped wearing article (in the underpants).

Next, a predetermined amount of power is supplied to the sweating mannequin to raise the body surface temperature. In the present embodiment, the power was supplied at 120.6 W/h. Further, liquid (sweat) is discharged at a rate of 100 g/m²/h from the plurality of sweat pores of the sweating mannequin. In the present embodiment, water is used as a liquid to be discharged.

Next, the measurement of the temperature at the temperature measurement positions is started after one minute has elapsed from the start of the power supply, and the temperature measurement is performed over time until a predetermined time period has elapsed. In the present inside-underpants-temperature measuring experiment, in consideration of the wearing time of the diaper, the temperature measurement was performed for at least 60 minutes from the start of the power supply. It should be noted that the temperature measurement is performed independently at the temperature measurement positions located on the front side and the temperature measurement positions located on the back side. The average value of the temperatures measured at the two temperature measurement positions on the front side (see FIG. 11A) is defined as the inside-underpants temperature on the front side. Similarly, the average value of the temperatures measured at the two temperature measurement positions on the back side (see FIG. 11B) is defined as the inside-underpants temperature on the back side.

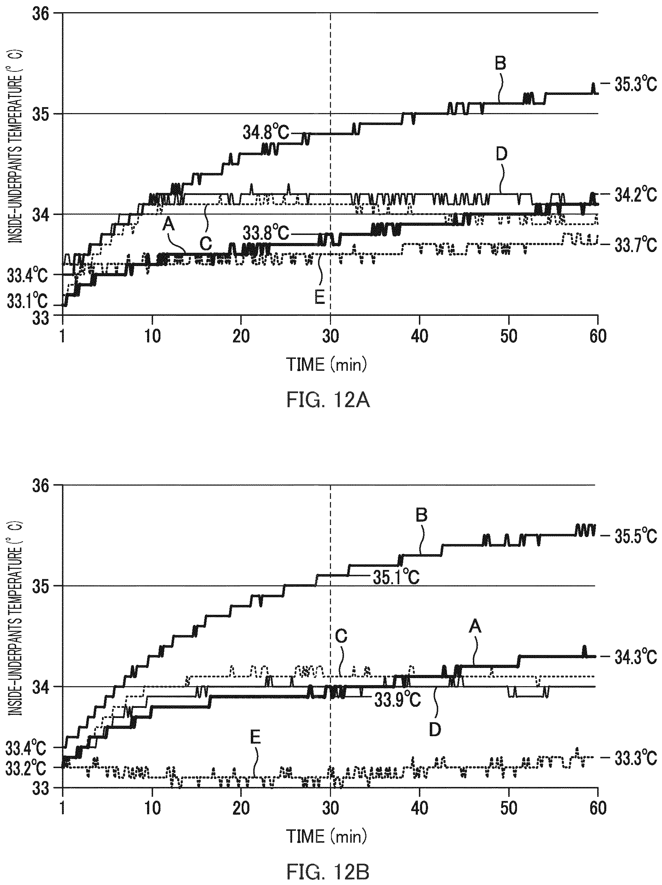

FIGS. 12A and 12B are diagrams showing the results of measurement of the inside-underpants temperature for several types of underpants-shaped wearing article. FIG. 12A shows the changes of the inside-underpants temperature over time on the front side, and FIG. 12B shows the changes of the inside-underpants temperature over time on the back side.

In FIGS. 12A and 12B, the measurement result of the inside-underpants temperature when the "diaper 1" according to the present embodiment is put on the sweating mannequin is shown as Example A. That is, Example A is temperature data measured in a state where the temperature measurement position described in FIG. 11 is covered with the diaper 1. Hereinafter, the temperature measured in a state where the temperature measurement position is covered with the diaper 1 will also be referred to as a "first temperature".

Further, as comparative examples with respect to Example A, the results of measuring the inside-underpants temperature when three types of underpants-shaped wearing article different from that of the diaper 1 are put on the sweating mannequin are shown as Comparative Examples B to D. The underpants-shaped wearing article of Comparative Example B is an underpants-shaped diaper in which a hydrophilic nonwoven fabric is not used. Examples of the underpants-shaped diaper of Comparative Example B include a diaper 1 in which the non-skin-side sheets 32 and 42 that constitute the waist member 20 are changed from a hydrophilic nonwoven fabric to a hydrophobic nonwoven fabric. However, an underpants-shaped diaper having another shape may be used as long as an underpants-shaped diaper in which a hydrophilic nonwoven fabric is not used.

The underpants-shaped wearing article of Comparative Example C is a common cotton underpants. Examples of the cotton underpants of Comparative Example C include commercially available underwear (not shown) containing 100% cotton. The underpants-shaped wearing article of Comparative Example D is general synthetic fiber underpants. Examples of the synthetic fiber underpants of Comparative Example D include commercially available underwear (not shown) containing, for example, 92% of nylon, 6% of polyurethane, and 2% of cotton.

Further, in FIGS. 12A and 12B, as Comparative Example E, there is shown the result of measuring the changes in temperature over time in a state where nothing is put on the sweating mannequin. That is, Comparative Example E is data of the temperature measured in a state where the temperature measurement position illustrated in FIG. 11 is not covered with anything and shows the body surface temperature itself of the sweating mannequin. It should be noted that in the following, the temperature measured in a state where the temperature measurement position is not covered is referred to as a "second temperature".

In the case where the temperature is measured without putting anything on the sweating mannequin (Comparative Example E), the body surface temperature (second temperature) gradually rises after the start of the power supply to the sweating mannequin. The front side body surface temperature (second temperature) of the sweating mannequin at the time of the start of the temperature measurement in one minute has elapsed after the start of the power supply is 33.1°C (see FIG. 12A). Similarly, the back side body surface temperature (second temperature) of the sweating mannequin at the time of the start of the temperature measurement is 33.2°C (see FIG. 12B).

When the power is continuously supplied in this state, the front side body surface temperature of the Comparative Example E gradually rises with the lapse of time, and the front side body surface temperature (second temperature) after 60 minutes have elapsed is 33.7°C (see FIG. 12A). Further, the back side body surface temperature (second temperature) after 60 minutes has elapsed is 33.3°C (see FIG. 12B). It should be noted that, in general, the average skin temperature (surface temperature) in a range of 33°C to 34°C is considered to be a comfortable temperature at which a human feel neither hot nor cold (Mayu Nonaka et al., "Differences between Mean and Local skin Temperatures with Neutral Thermal Sensation and Thermal Comfort : A comparison of skin temperature differences among female students between in summer and in winter", Journal of human and living environment 16(2), 91 to 97, 2009).

In contrast, in the case where the inside-underpants temperature was measured in a state where the diaper 1 was put on the sweating mannequin (Example A), the front inside-underpants temperature (first temperature) of the sweating mannequin at the time of the start of the temperature measurement was 33.1°C, and the back inside-underpants temperature (first temperature) was 33.2°C. That is, at the time of the start of the temperature measurement, there is no clear temperature difference between the first temperature (Example A) measured in a state where the diaper 1 is put on and the second temperature (comparative example E) measured in a state where nothing is put on.

Further, in Example A, the inside-underpants temperature gradually rises with the lapse of time, and the front inside-underpants temperature (first temperature) after 60 minutes have elapsed is 34.2°C (see FIG. 12A) and the back inside-underpants temperature (first temperature) after 60 minutes have elapsed is 34.3°C (see FIG. 12B). That is, it can be seen that, at the time point when approximately 60 minutes has elapsed from the start of the measurement (accurately, at the time point when 60 minutes has elapsed from the start of the power supply), the first temperature measured in a state where the diaper 1 is put on (Example A) is 0.5°C to 1.0°C higher than the second temperature measured in a state where nothing is put on (Comparative Example E).

The temperature rise (0.5°C to 1.0°C) in Example A is at approximately the same level as the temperature rise in Comparative Example C and Comparative Example D (see FIGS. 12A and 12B). That is, it was clarified that the temperature rise when the diaper 1 of the present embodiment was put on was almost the same as that of commercially available cotton underpants or synthetic fiber underpants. Further, the first temperature of 34.2°C to 34.3°C after 60 minutes have elapsed is not significantly shifted from the comfortable temperature (33°C to 34°C) that a human does not feel hot or cold. Therefore, the temperature rise when the diaper 1 is put on is approximately the same as the temperature rise while general cloth underwear is put on (Comparative Examples C and D). Accordingly, it can make the wearer less likely to feel unpleasantness or discomfort.

It should be noted that there is a possibility that an error of approximately ±0.5°C may occur in the measurement of the inside-underpants temperature measurement. However, even in the case where the error becomes maximum, a difference between the front and back inside-underpants temperature of the diaper 1 (first temperature) and the temperature in a state where nothing is put on (second temperature) is 1.5°C or less.

On the other hand, in the underpants-shaped diaper of Comparative Example B in which the hydrophilic nonwoven fabric is not used, the front inside-underpants temperature and the back inside-underpants temperature after 60 minutes have elapsed from the start of the power supply are 35.3°C and 35.5°C, respectively, and the difference from the temperature in a state where nothing is put on (Comparative Example E) is larger than 1.5°C. Therefore, it can be seen that the temperature rise of the diaper 1 is kept lower than that of the underpants-shaped diaper of Comparative Example B.

This is because, in the waist member 20 of the diaper 1, the heat of vaporization is likely to be removed by evaporating the moisture that has been absorbed by the hydrophilic nonwoven fabric, into the atmosphere from the non-skin side of the waist member 20. Accordingly, a difference between the inside-underpants temperature in a state where the diaper 1 is put on (Example A) and the temperature in a state where the diaper is not put on (Comparative Example E) (temperature rise) is suppressed to 1.5°C or less, and heat is less likely to be accumulated inside the diaper, suppressing excessive temperature rise. This can make the wearer of the diaper 1 less likely to feel discomfort.

Further, the temperature measured in a state where the temperature measurement position is covered with the underpants-shaped diaper of Comparative Example B in which the hydrophilic nonwoven fabric is not used is referred to as a "third temperature". After 60 minutes have elapsed from the start of the power supply, a difference between the third temperature (Comparative Example B) and the first temperature (Example A) is 1°C or more. Specifically, in the front side of the sweating mannequin, the third temperature is 35.3°C after 60 minutes have elapsed, whereas the first temperature is 34.2°C after 60 minutes have elapsed, and the third temperature in Comparative Example B is 1.1 C higher than the first temperature in Example A (see FIG. 12A). Similarly, in the back side of the sweating mannequin, the third temperature is 35.5°C after 60 minutes have elapsed, whereas the first temperature is 34.3°C after 60 minutes have elapsed, and the third temperature in Comparative Example B is 1.2°C higher than the first temperature in Example A (see FIG. 12B).

As described above, in the diaper 1 of the present embodiment (Example A), it can be seen that the temperature rise after 60 minutes have elapsed is at least 1°C or more low compared with the underpants-shaped diaper in which the hydrophilic nonwoven fabric is not used (Comparative Example B). As described above, the underpants-shaped diaper of Comparative Example B and the underpants-shaped diaper (diaper 1) of Example A differ only in the presence of the hydrophilic nonwoven fabric. Therefore, it became clear that, by providing the hydrophilic nonwoven fabric to facilitate evaporation of moisture such as sweat into the atmosphere, the temperature rise was more likely to be suppressed compared with the conventional underpants-shaped disposable diaper formed of the hydrophobic nonwoven fabric.

Further, the diaper 1 of Example A and the underpants-shaped diaper of Comparative Example B have different behaviors at the time of temperature rise. In FIG. 12A, the first temperature (Example A) is 33.1°C at the time point of the start of the measurement and rises to 33.8°C after 30 minutes have elapsed. That is, the temperature rise rate of the first temperature per unit time calculated based on the temperature measurement data for 29 minutes from the start of measurement is (33.8 - 33.1) x 60/29 ≈ 1.45 °C/h. On the other hand, the third temperature (Comparative Example B) is 33.4°C at the time point of the start of the measurement and rises to 34.8°C after 30 minutes have elapsed. That is, the temperature rise rate of the third temperature per unit time calculated based on the temperature measurement data for 29 minutes from the start of measurement is (34.8 - 33.4) x 60/29 ≈ 2.90 °C/h. Therefore, the temperature rise rate per unit time of the first temperature on the front side at a time point when 30 minutes have elapsed from the start of the power supply is 0.5 times (= 1.45/2.90) the temperature rise rate per unit time of the third temperature.

Similarly, in FIG. 12B, the first temperature (Example A) is 33.2°C at the time point of the start of the measurement and rises to 33.9°C after 30 minutes have elapsed. That is, the temperature rise rate of the first temperature per unit time calculated based on the temperature measurement data for 29 minutes from the start of measurement is (33.9 - 33.2) x 60/29 ≈ 1.45 °C/h. On the other hand, the third temperature (Comparative Example B) is 33.4°C at the time point of the start of the measurement and rises to 35.1°C after 30 minutes have elapsed. That is, the temperature rise rate of the third temperature per unit time calculated based on the temperature measurement data for 29 minutes from the start of measurement is (35.1 - 33.4) x 60/29 ≈ 3.52 °C/h. Therefore, the temperature rise rate per unit time of the first temperature on the back side at a time point when 30 minutes have elapsed from the start of the power supply is 0.42 times (= 1.45/3.52) the temperature rise rate per unit time of the third temperature.

From such a result, it can be seen that in the diaper 1 of the present embodiment (Example A), the temperature rise rate at the time point when 30 minutes have elapsed is 1/2 or less compared with the underpants-shaped diaper in which the hydrophilic nonwoven fabric is not used (Comparative Example B). Accordingly, it is possible to confirm that, in the diaper 1 of the present embodiment, the inside-underpants temperature is less likely to rise compared with the conventional underpants-shaped diaper. Further, in the diaper 1, it can be seen that the temperature increase in approximately 30 minutes from the start of the measurement of the internal temperature in the underpants is kept low, and the diaper 1 has an excellent feeling of use immediately after putting on the diaper 1.

Further, the temperature measurement positions (predetermined position) in the present inside-underpants-temperature measuring experiment are arranged at a positions on both the front side and the back side which are covered with the waist member 20 (the front waist portion 30 and the back waist portion 40) (see FIGS. 11A and 11B). In other words, in a state where the diaper 1 is put on, the temperature of the position where the wearer's body is covered with the waist member 20 is measured. Further, it was clarified that in the diaper 1, the temperature rise at the position covered with the waist member 20 was reduced compared with the diaper of Comparative Example B. Therefore, by putting on the diaper 1, in the front and back waist regions in which the wearer is more likely to sweat, it makes it easier to prevent the inside-underpants temperature from becoming excessively high or stuffiness inside the underpants. This can make the wearer less likely to feel discomfort.

As described above, in the diaper 1 of the present embodiment, it was clarified that the effect of the hydrophilic nonwoven fabric providing in the waist member 20 promote the absorption and evaporation of moisture, making it possible to suppress the internal temperature rise (inside-underpants temperature) of the diaper 1. Therefore, according to the diaper 1 of the present embodiment, it is possible to realize an underpants-shaped absorbent article which can make the wearer less likely to feel discomfort.

### Second Embodiment

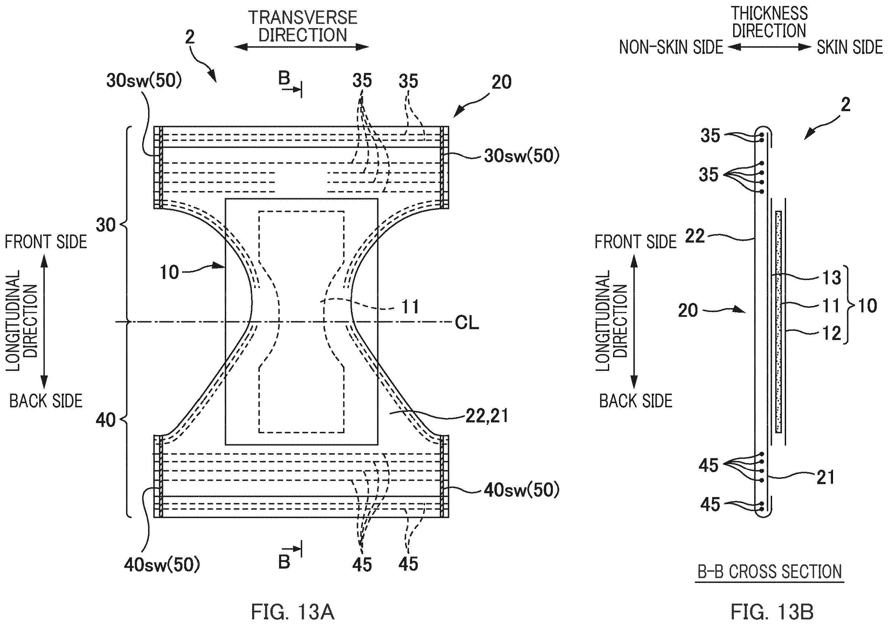

In a second embodiment, an underpants-shaped diaper 2 (hereinafter, also referred to as a "diaper 2") having a configuration partially different from that of the first embodiment will be described. FIG. 13A is a plan view of the diaper 2 in the unfolded and stretched state. FIG. 13B is a schematic cross-sectional view taken along a line B-B in FIG. 13A. It should be noted that the directions (e.g., the longitudinal direction, the transverse direction, or the like) in FIGS. 13A and 13B is the same as the directions in the first embodiment.

The diaper 2 of the second embodiment has the liquid-absorbent absorbent main body 10, and the waist member 20 that is arranged on the non-skin side of the absorbent main body 10. However, in the diaper 2, the front waist portion 30 and the back waist portion 40 are integrally constituted. That is, the diaper 2 is a so-called two-piece type disposable diaper formed of the absorbent main body 10 serving as an interior body and the waist member 20 serving as an exterior body.

When shaping the diaper 2 in the unfolded state in FIG. 13A into an underpants shape, the absorbent main body 10 and the waist member 20 are folded one time at a fold position, which is the longitudinal central position CL. In this folded state, the front waist portion 30 and the back waist portion 40 that face each other are joined and connected to each other at two lateral side portions 30sw and 40sw to form the pair of side joining portions 50 and 50. Accordingly, similar to the diaper 1 in FIG. 1, the diaper 2 is in an underpants-shaped state having a waist opening BH and a pair of leg openings LH and LH.

In the diaper 2, the basic configuration and function of the absorbent main body 10 are substantially the same as those of the absorbent main body 10 of the diaper 1 of the first embodiment, and therefore description thereof is omitted. It should be noted that, in FIG. 13A, the crotch portion including the central position CL in the longitudinal direction (the lengthwise direction of the absorbent main body 10) has a portion in which the lateral width of the absorbent main body 10 is wider than the lateral width of the waist member 20. Therefore, during usage of the diaper 1, in the region in the vicinity of the wearer's crotch portion, two lateral end edge portions (edges) of the absorbent main body 10 come into contact with the wearer's legs.

Further, in the diaper 2, a portion of the waist member 20 located on the front side with respect to the central position CL in the longitudinal direction (the lengthwise direction of the absorbent main body 10) serves as the front waist portion 30, and a portion located on the back side with respect to the central position CL serves as the back waist portion 40. The waist member 20 includes: a skin-side sheet 21; a non-skin-side sheet 22 that is overlaid to be adjacent to the non-skin side of the skin-side sheet 21; and waist elastic members 35 and 45 that are arranged between the skin-side sheet 21 and the non-skin-side sheet 22 in the thickness direction (see FIG. 13B).

The skin-side sheet 21 is a hydrophobic nonwoven fabric sheet similar to the skin-side sheets 31 of the diaper 1. The non-skin-side sheet 22 is a hydrophilic nonwoven fabric similar to the non-skin-side sheets 32 of the diaper 1. That is, the non-skin-side sheet 22 is formed of nonwoven fabric having higher hydrophilicity than the skin-side sheet 21. Then, similar to the diaper 1, the waist elastic members 35 and 45 are formed of elastic strings or the like, and are attached between the skin-side sheet 21 and the non-skin-side sheet 22 in a state of being stretched in the lateral direction.

Also in the diaper 2 of the second embodiment, as in the diaper 1 of the first embodiment, moisture can be absorbed from the wearer's skin and the absorbed moisture can be efficiently evaporated into the atmosphere. Specifically, the hydrophobic nonwoven fabric (skin-side sheet 21) arranged so as to be in contact with the wearer's skin contracts accompanying with the contraction of the waist elastic members 35 and 45, and thereby the inter-fiber distance between the fibers that constitute the hydrophobic nonwoven fabric is reduced. Accordingly, the capillary phenomenon is likely to occur, and moisture such as sweat is absorbed from the wearer's skin. When the waist elastic members 35 and 45 are stretched, it increases the contact area between the hydrophobic nonwoven fabric (skin-side sheet 21) and the hydrophilic nonwoven fabric (non-skin-side sheet 22). Consequently, the moisture is transferred from the hydrophobic nonwoven fabric having low hydrophilicity, to the hydrophilic nonwoven fabric having high hydrophilicity. The transferred moisture is evaporated into the atmosphere from the interface between the hydrophilic nonwoven fabric and the atmosphere. In this manner, moisture is efficiently released from the wearer's skin to the atmosphere, and this can make the wearer less likely to feel discomfort.

Further, in the diaper 2, similarly to the diaper 1, the moisture absorbed from the wearer's skin can be efficiently evaporated into the atmosphere. Therefore, as illustrated in FIG. 12, it is possible to suppress the rising of the inside-underpants temperature during usage of the diaper. This can make the wearer less likely to feel discomfort during usage of the diaper 2.

### Third Embodiment

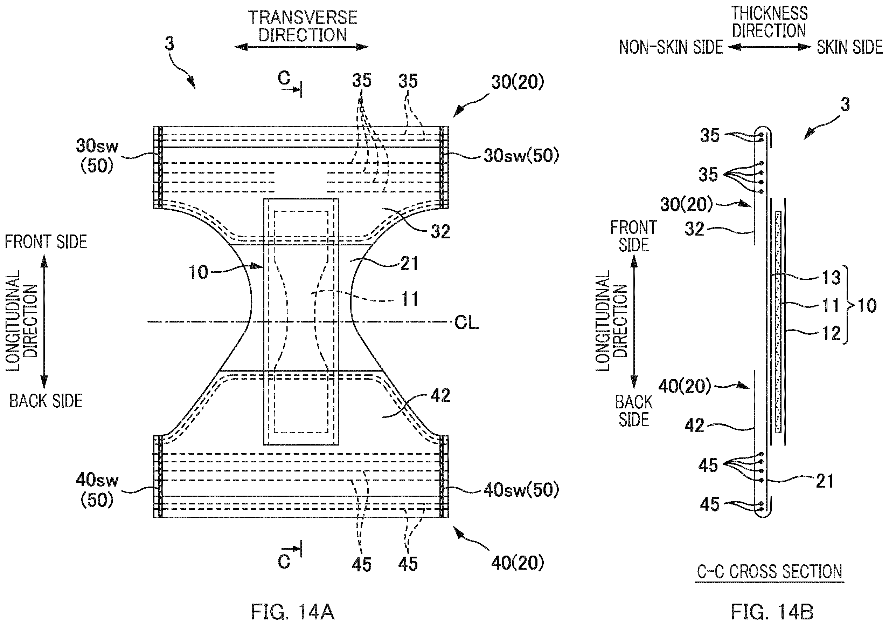

In a third embodiment, an underpants-shaped diaper 3 (hereinafter, also referred to as a "diaper 3") having a configuration partially different from those of the first embodiment and the second embodiment will be described. FIG. 14A is a plan view of the diaper 3 in the unfolded and stretched state. FIG. 14B is a schematic cross-sectional view taken along a line C-C in FIG. 14A. It should be noted that the directions (e.g., the longitudinal direction, the transverse direction, or the like) in FIGS. 14A and 14B is the same as the directions in the first embodiment.

The diaper 3 of the third embodiment has the liquid-absorbent absorbent main body 10, and the waist member 20 that is arranged on the non-skin side of the absorbent main body 10. However, in the diaper 3, as shown in FIG. 14B, a part of the member that constitutes the front waist portion 30 (skin-side sheet 21) and a part of the member that constitutes the back waist portion 40 (skin-side sheet 21) are integrally constituted. Hereinafter, a diaper having such a structure will also be referred to as a simple three-piece type disposable diaper.

When shaping the diaper 3 in the unfolded state in FIG. 14A into an underpants shape, the absorbent main body 10 and the waist member 20 are folded one time at a fold position, which is the longitudinal central position CL. In this folded state, the front waist portion 30 and the back waist portion 40 that face each other are joined and connected to each other at two lateral side portions 30sw and 40sw to form the pair of side joining portions 50 and 50. Accordingly, similar to FIG. 1, the diaper 3 is in an underpants-shaped state having a waist opening BH and a pair of leg openings LH and LH.

In the diaper 3, the basic configuration and function of the absorbent main body 10 are substantially the same as those of the absorbent main body 10 of the diaper 1 of the first embodiment, and therefore description thereof is omitted.

Further, in the diaper 3, a portion of the waist member 20 located on the front side with respect to the central position CL in the longitudinal direction (the lengthwise direction of the absorbent main body 10) serves as the front waist portion 30, and a portion located on the back side with respect to the central position CL serves as the back waist portion 40. The front waist portion 30 includes: the single skin-side sheet 21 that is located on the skin side in the thickness direction and extends from the one end side (front side) to the other end side (back side) in the longitudinal direction; the non-skin-side sheet 32 that is located on the front side in the longitudinal direction and is overlaid to be adjacent to the non-skin side of the skin-side sheet 21; and the waist elastic members 35 that are arranged between the skin-side sheet 21 and the non-skin-side sheet 32 in the thickness direction. The back waist portion 40 includes: the skin-side sheet 21 that is common to the front waist portion 30; the non-skin-side sheet 42 that is located on the back side in the longitudinal direction and is overlaid to be adjacent to the non-skin side of the skin-side sheet 21; and the waist elastic members 45 that are arranged between the skin-side sheet 21 and the non-skin-side sheet 42 in the thickness direction.

The skin-side sheet 21 is a hydrophobic nonwoven fabric sheet similar to the skin-side sheets 31 of the diaper 1. The non-skin-side sheets 32 and 42 are hydrophilic nonwoven fabrics similar to the non-skin-side sheets 32 and 42 of the diaper 1. That is, the non-skin-side sheets 32 and 42 are each formed of a nonwoven fabric sheet having higher hydrophilicity than the skin-side sheet 21. Then, similar to the diaper 1, the waist elastic members 35 and 45 are formed of elastic strings or the like, and are attached between the skin-side sheet 21 and the non-skin-side sheets 32 and 42 in a state of being stretched in the lateral direction.

Also in the diaper 3 of the third embodiment, as in the diaper 1 of the first embodiment, moisture can be absorbed from the wearer's skin and the absorbed moisture can be efficiently evaporated into the atmosphere. Specifically, the hydrophobic nonwoven fabric (skin-side sheet 21) arranged so as to be in contact with the wearer's skin contracts accompanying with the contraction of the waist elastic members 35 and 45, and thereby the inter-fiber distance between the fibers that constitute the hydrophobic nonwoven fabric is reduced. Accordingly, the capillary phenomenon is likely to occur, and moisture such as sweat is absorbed from the wearer's skin. When the waist elastic members 35 and 45 are stretched, it increases the contact area between the hydrophobic nonwoven fabric (skin-side sheet 21) and the hydrophilic nonwoven fabric (non-skin-side sheets 32 and 42). Consequently, the moisture is transferred from the hydrophobic nonwoven fabric having low hydrophilicity, to the hydrophilic nonwoven fabric having high hydrophilicity. The transferred moisture is evaporated into the atmosphere from the interface between the hydrophilic nonwoven fabric and the atmosphere. In this manner, moisture is efficiently released from the wearer's skin to the atmosphere, and this can make the wearer less likely to feel discomfort.

Further, in the diaper 3, similarly to the diaper 1 and the diaper 2, the moisture absorbed from the wearer's skin can be efficiently evaporated into the atmosphere. Therefore, as illustrated in FIG. 12, it is possible to suppress the rising of the inside-underpants temperature during usage of the diaper. This can make the wearer less likely to feel discomfort during usage of the diaper 3.

### Other Embodiments

Although the above embodiments of the present invention have been described, but the above-described embodiments are intended to facilitate the understanding of the present invention and are not intended to limit the interpretation of the present invention. In addition, the present invention can be modified or improved within the scope of the present invention.

### Reference Signs List

1: diaper (underpants-shaped absorbent article) (first embodiment)
2: diaper (underpants-shaped absorbent article) (second embodiment),
3: diaper (underpants-shaped absorbent article) (third embodiment),
10: absorbent main body,
11: absorbent core, 11b: core-wrapping sheet, 11c: narrow portion,
12: top sheet,
13: back sheet, 13a: liquid-impermeable sheet, 13b: exterior sheet,
15: leak-proof wall portion, 16: leak-proof-wall elastic member,
17: leg elastic member,
20: waist member,
21: skin-side sheet (hydrophobic nonwoven fabric)
22: non-skin-side sheet (hydrophilic nonwoven fabric),
30: front waist portion,
30sw: side portion,
31: skin-side sheet (hydrophobic nonwoven fabric)
32: non-skin-side sheet (hydrophilic nonwoven fabric), 32f: folded-back portion,
32h: hole portion, 32hs: side wall portion, 32hp: protruding portion,
32s: non-skin-side surface
35: waist elastic member,
36: skin surface sheet,
40: back waist portion, 40b: crotch region,
40sw: side portion,
41: skin-side sheet (hydrophobic nonwoven fabric)
42: non-skin-side sheet (hydrophilic nonwoven fabric), 42f: folded-back portion,
42h: hole portion,
45: waist elastic member,
46: skin surface sheet, 47: curved elastic member,
50: side joining portion,
60: welding portion,
60s: welding portion pair,
BH: waist opening, LH: leg opening,
CL: central position (longitudinal direction, lengthwise direction)

## Claims

1. An underpants-shaped absorbent article (1, 2, 3) having a vertical direction, a lateral direction, and a front-back direction that intersect with each other,
the underpants-shaped absorbent article (1, 2, 3) comprising:
a liquid-absorbent absorbent main body (10); and
a waist member (20) that is provided on a non-skin side with respect to the absorbent main body (10),
an at least partial region of the waist member (20) having
a hydrophobic nonwoven fabric (31) that is arranged farthest on a skin side,
a hydrophilic nonwoven fabric (32)
that is overlaid adjacent to a non-skin side of the hydrophobic nonwoven fabric (31) and
that has higher hydrophilicity than the hydrophobic nonwoven fabric (31), and
an elastic member (35)
that is provided between the hydrophobic nonwoven fabric (31) and the hydrophilic nonwoven fabric (32) and
that stretches and contracts in the lateral direction, **characterized in that**
an average fiber density of the hydrophobic nonwoven fabric (31) is larger than an average fiber density of the hydrophilic nonwoven fabric (32) the average fiber density being measured using the method specified in the description.

2. The underpants-shaped absorbent article (1, 2, 3) according to claim 1, wherein
a portion in which the hydrophobic nonwoven fabric (31) and the hydrophilic nonwoven fabric (32) are joined is provided around at least the elastic member (35).

3. The underpants-shaped absorbent article (1, 2, 3) according to claim 2, wherein
the hydrophobic nonwoven fabric (31) and the hydrophilic nonwoven fabric (32) are joined with an adhesive via the elastic member (35).

4. The underpants-shaped absorbent article (1, 2, 3) according to any one of claims 1 to 3, wherein
the waist member (20) has a front waist member (30) and a back waist member (40),
the waist member (20) has a pair of side joining portions (50) that join the front waist member (30) and the back waist member (40) in two lateral end portions, and
the at least partial region of the waist member (20) is provided in any portion between the pair of side joining portions (50) in the lateral direction.

5. The underpants-shaped absorbent article (1, 2, 3) according to claim 4, wherein
the at least partial region of the waist member (20) is provided in the back waist member (40).

6. The underpants-shaped absorbent article (1, 2, 3) according to any one of claims 1 to 5, wherein
at least a part of the hydrophilic nonwoven fabric (32) is arranged farthest on a non-skin side of the waist member (20).

7. The underpants-shaped absorbent article (1, 2, 3) according to any one of claims 1 to 6, wherein
the hydrophobic nonwoven fabric (31) has a plurality of compressed portions in which the hydrophobic nonwoven fabric (31) is compressed in a thickness direction.

8. The underpants-shaped absorbent article (1, 2, 3) according to any one of claims 1 to 7, wherein
the hydrophilic nonwoven fabric (32) has a plurality of hole portions (32h) that penetrate the hydrophilic nonwoven fabric (32) in a thickness direction.

9. The underpants-shaped absorbent article (1, 2, 3) according to claim 8, wherein
an average thickness of the hydrophilic nonwoven fabric (32) is larger than an average thickness of the hydrophobic nonwoven fabric (31) the average thickness being measured with the method specified in the description.

10. An underpants-shaped absorbent article (1, 2, 3) according to claim 8 or 9, wherein a skin-side surface of the hydrophilic nonwoven fabric (32) has a protruding portion (32hp)in which an outer edge portion of the hole portion (32h) protrudes toward a skin side.

11. The underpants-shaped absorbent article (1, 2, 3) according to any one of claims 8 to 10, wherein
the underpants-shaped absorbent article (1, 2, 3) has a portion where the hole portion (32h) and the elastic member (35) overlap each other.

12. The underpants-shaped absorbent article (1, 2, 3) according to any one of claims 8 to 11, wherein
the plurality of hole portions (32h) are provided between two elastic members (35) that are arranged adjacent to each other in the vertical direction.

13. The underpants-shaped absorbent article (1, 2, 3) according to any one of claims 8 to 12, wherein
the hydrophobic nonwoven fabric (31) does not have a through hole that penetrates the hydrophobic nonwoven fabric (31) in the thickness direction.

14. The underpants-shaped absorbent article (1, 2, 3) according to any one of claims 1 to 13, wherein
an average basis weight of the hydrophilic nonwoven fabric (32) is larger than an average basis weight of the hydrophobic nonwoven fabric (31) the average basis weight being measured with the method specified in the description.

## Patentansprüche

1. Unterhosen-förmiger absorbierender Artikel (1, 2, 3), der eine vertikale Richtung, eine laterale Richtung und eine Vom-Hinten-Richtung aufweist, die einander schneiden,
wobei der Unterhosen-förmige absorbierende Artikel (1, 2, 3) Folgendes umfasst:
einen flüssigkeitsabsorbierenden absorbierenden Hauptkörper (10); und
ein Taillenelement (20), das auf einer Nicht-Hautseite in Bezug auf den absorbierenden Hauptkörper (10) bereitgestellt ist,
wobei ein mindestens Teilbereich des Taillenelements (20) Folgendes aufweist:
einen hydrophoben Vliesstoff (31), der am weitesten auf einer Hautseite angeordnet ist,
einen hydrophilen Vliesstoff (32),
der angrenzend an eine Nicht-Hautseite des hydrophoben Vliesstoffs (31) überlagert und
der eine höhere Hydrophilie aufweist als der hydrophobe Vliesstoff (31),
und
ein elastisches Element (35),
das zwischen dem hydrophoben Vliesstoff (31) und dem hydrophilen Vliesstoff (32) bereitgestellt ist und
das sich in der lateralen Richtung dehnt und zusammenzieht,
**dadurch gekennzeichnet, dass**
eine durchschnittliche Faserdichte des hydrophoben Vliesstoffs (31) größer ist als eine durchschnittliche Faserdichte des hydrophilen Vliesstoffs (32), wobei die durchschnittliche Faserdichte unter Verwendung des Verfahrens, das in der Beschreibung aufgeführt ist, gemessen wird.

2. Unterhosen-förmiger absorbierender Artikel (1, 2, 3) nach Anspruch 1, wobei
ein Teil, in dem der hydrophobe Vliesstoff (31) und der hydrophile Vliesstoff (32) verbunden sind, um zumindest das elastische Element (35) bereitgestellt ist.

3. Unterhosen-förmiger absorbierender Artikel (1, 2, 3) nach Anspruch 2, wobei
der hydrophobe Vliesstoff (31) und der hydrophile Vliesstoff (32) mit einem Haftmittel über das elastische Element (35) verbunden sind.

4. Unterhosen-förmiger absorbierender Artikel (1, 2, 3) nach einem der Ansprüche 1 bis 3, wobei
das Taillenelement (20) ein vorderes Taillenelement (30) und ein hinteres Taillenelement (40) aufweist,
das Taillenelement (20) ein Paar von Seitenverbindungsteilen (50) aufweist, die das vordere Taillenelement (30) und das hintere Taillenelement (40) in zwei lateralen Endteilen verbinden, und
der mindestens Teilbereich des Taillenelements (20) in jedwedem Teil zwischen dem Paar von Seitenverbindungsteilen (50) in der lateralen Richtung bereitgestellt ist.

5. Unterhosen-förmiger absorbierender Artikel (1, 2, 3) nach Anspruch 4, wobei
der mindestens Teilbereich des Taillenelements (20) in dem hinteren Taillenelement (40) bereitgestellt ist.

6. Unterhosen-förmiger absorbierender Artikel (1, 2, 3) nach einem der Ansprüche 1 bis 5, wobei
zumindest ein Teil des hydrophilen Vliesstoffs (32) am weitesten auf einer Nicht-Hautseite des Taillenelements (20) angeordnet ist.

7. Unterhosen-förmiger absorbierender Artikel (1, 2, 3) nach einem der Ansprüche 1 bis 6, wobei
der hydrophobe Vliesstoff (31) eine Vielzahl von komprimierten Teilen aufweist, in denen der hydrophobe Vliesstoff (31) in einer Dickenrichtung komprimiert ist.

8. Unterhosen-förmiger absorbierender Artikel (1, 2, 3) nach einem der Ansprüche 1 bis 7, wobei
der hydrophile Vliesstoff (32) eine Vielzahl von Lochteilen (32h) aufweist, die den hydrophilen Vliesstoff (32) in einer Dickenrichtung durchdringen.

9. Unterhosen-förmiger absorbierender Artikel (1, 2, 3) nach Anspruch 8, wobei
eine durchschnittliche Dicke des hydrophilen Vliesstoffs (32) größer ist als eine durchschnittliche Dicke des hydrophoben Vliesstoffs (31), wobei die durchschnittliche Dicke mit dem Verfahren, das in der Beschreibung aufgeführt ist, gemessen wird.

10. Unterhosen-förmiger absorbierender Artikel (1, 2, 3) nach Anspruch 8 oder 9, wobei eine Hautseitenoberfläche des hydrophilen Vliesstoffs (32) einen vorstehenden Teil (32hp) aufweist, in dem ein Außenrandteil des Lochteils (32h) in Richtung einer Hautseite vorsteht.

11. Unterhosen-förmiger absorbierender Artikel (1, 2, 3) nach einem der Ansprüche 8 bis 10, wobei
der Unterhosen-förmige absorbierende Artikel (1, 2, 3) einen Teil aufweist, wo der Lochteil (32h) und das elastische Element (35) miteinander überlappen.

12. Unterhosen-förmiger absorbierender Artikel (1, 2, 3) nach einem der Ansprüche 8 bis 11, wobei
die Vielzahl von Lochteilen (32h) zwischen zwei elastischen Elementen (35) bereitgestellt ist, die in der vertikalen Richtung angrenzend aneinander angeordnet sind.

13. Unterhosen-förmiger absorbierender Artikel (1, 2, 3) nach einem der Ansprüche 8 bis 12, wobei
der hydrophobe Vliesstoff (31) kein Durchgangsloch aufweist, das den hydrophoben Vliesstoff (31) in der Dickenrichtung durchdringt.

14. Unterhosen-förmiger absorbierender Artikel (1, 2, 3) nach einem der Ansprüche 1 bis 13, wobei
ein durchschnittliches Flächengewicht des hydrophilen Vliesstoffs (32) größer ist als ein durchschnittliches Flächengewicht des hydrophoben Vliesstoffs (31), wobei das durchschnittliche Flächengewicht mit dem Verfahren, das in der Beschreibung aufgeführt ist, gemessen wird.

## Revendications

1. Article absorbant en forme de culotte (1, 2, 3) ayant une direction verticale, une direction latérale et une direction allant d'avant en arrière qui se croisent les unes les autres,
l'article absorbant en forme de culotte (1, 2, 3) comportant :
un corps principal absorbant qui absorbe les liquides (10) ; et
un élément au niveau de la taille (20) qui est mis en œuvre sur un côté non orienté vers la peau par rapport au corps principal absorbant (10),
une région au moins partielle de l'élément au niveau de la taille (20) ayant
un tissu non tissé hydrophobe (31) qui est agencé au plus loin sur un côté orienté vers la peau,
un tissu non tissé hydrophile (32)
qui est superposé de manière adjacente par rapport à un côté non orienté vers la peau du tissu non tissé hydrophobe (31) et
qui a une hydrophilie supérieure par rapport à celle du tissu non tissé hydrophobe (31), et
un élément élastique (35)
qui est mis en œuvre entre le tissu non tissé hydrophobe (31) et le tissu non tissé hydrophile (32) et
qui s'étire et se contracte dans la direction latérale, **caractérisé en ce que**
une densité moyenne de fibres du tissu non tissé hydrophobe (31) est supérieure à une densité moyenne de fibres du tissu non tissé hydrophile (32), la densité moyenne de fibres étant mesurée à l'aide du procédé spécifié dans la description.

2. Article absorbant en forme de culotte (1, 2, 3) selon la revendication 1, dans lequel
une partie dans laquelle le tissu non tissé hydrophobe (31) et le tissu non tissé hydrophile (32) sont joints est mise en œuvre au moins autour de l'élément élastique (35).

3. Article absorbant en forme de culotte (1, 2, 3) selon la revendication 2, dans lequel
le tissu non tissé hydrophobe (31) et le tissu non tissé hydrophile (32) sont joints au moyen d'un adhésif par le biais de l'élément élastique (35).

4. Article absorbant en forme de culotte (1, 2, 3) selon l'une quelconque des revendications 1 à 3, dans lequel
l'élément au niveau de la taille (20) a un élément avant au niveau de la taille (30) et un élément arrière au niveau de la taille (40),
l'élément au niveau de la taille (20) a une paire de parties d'assemblage latérales (50) qui assemblent l'élément avant au niveau de la taille (30) et l'élément arrière au niveau de la taille (40) dans deux parties d'extrémité latérales, et
la région au moins partielle de l'élément au niveau de la taille (20) est mise en œuvre dans n'importe quelle partie entre la paire de parties d'assemblage latérales (50) dans la direction latérale.

5. Article absorbant en forme de culotte (1, 2, 3) selon la revendication 4, dans lequel
la région au moins partielle de l'élément au niveau de la taille (20) est mise en œuvre dans l'élément arrière au niveau de la taille (40).

6. Article absorbant en forme de culotte (1, 2, 3) selon l'une quelconque des revendications 1 à 5, dans lequel
au moins une partie du tissu non tissé hydrophile (32) est agencé au plus loin sur un côté non orienté vers la peau de l'élément au niveau de la taille (20).

7. Article absorbant en forme de culotte (1, 2, 3) selon l'une quelconque des revendications 1 à 6, dans lequel
le tissu non tissé hydrophobe (31) a une pluralité de parties comprimées dans lesquelles le tissu non tissé hydrophobe (31) est comprimé dans une direction allant dans le sens de l'épaisseur.

8. Article absorbant en forme de culotte (1, 2, 3) selon l'une quelconque des revendications 1 à 7, dans lequel
le tissu non tissé hydrophile (32) a une pluralité de parties formant trou (32h) qui pénètrent dans le tissu non tissé hydrophile (32) dans une direction allant dans le sens de l'épaisseur.

9. Article absorbant en forme de culotte (1, 2, 3) selon la revendication 8, dans lequel
une épaisseur moyenne du tissu non tissé hydrophile (32) est supérieure à une épaisseur moyenne du tissu non tissé hydrophobe (31), l'épaisseur moyenne étant mesurée à l'aide du procédé spécifié dans la description.

10. Article absorbant en forme de culotte (1, 2, 3) selon la revendication 8 ou la revendication 9, dans lequel
une surface orientée vers la peau du tissu non tissé hydrophile (32) a une partie faisant saillie (32hp) dans laquelle une partie formant bord extérieur de la partie formant trou (32h) fait saillie vers un côté orienté vers la peau.

11. Article absorbant en forme de culotte (1, 2, 3) selon l'une quelconque des revendications 8 à 10, dans lequel
l'article absorbant en forme de culotte (1, 2, 3) a une partie dans laquelle la partie formant trou (32h) et l'élément élastique (35) se chevauchent.

12. Article absorbant en forme de culotte (1, 2, 3) selon l'une quelconque des revendications 8 à 11, dans lequel
les parties de la pluralité de parties formant trou (32h) sont mises en œuvre entre deux éléments élastiques (35) qui sont agencés de manière adjacente l'un par rapport à l'autre dans la direction verticale.

13. Article absorbant en forme de culotte (1, 2, 3) selon l'une quelconque des revendications 8 à 12, dans lequel
le tissu non tissé hydrophobe (31) n'a pas de trou traversant qui pénètre dans le tissu non tissé hydrophobe (31) dans la direction allant dans le sens de l'épaisseur.

14. Article absorbant en forme de culotte (1, 2, 3) selon l'une quelconque des revendications 1 à 13, dans lequel
une masse surfacique moyenne du tissu non tissé hydrophile (32) est supérieure à une masse surfacique moyenne du tissu non tissé hydrophobe (31), la masse surfacique moyenne étant mesurée à l'aide du procédé spécifié dans la description.
